# EUROPEAN PATENT APPLICATION

(11) **EP 2 708 236 A1**
(43) Date of publication of application: **19.03.2014**
(21) Application number: 12184040.9
(22) Date of filing: 12.09.2012
(51) Int. Cl.: A61K 38/20, A61K 35/76, A61P 31/12, A61P 31/16, A61P 35/00

(54) **Tumor treatment**

(71) Applicant: Medizinische Universität Wien, 1090 Vienna (AT)
(72) Inventor: Bergmann, Michael, 1190 Vienna (AT); Weiss, Rene, 3131 Walpersdorf (AT)
(74) Representative: Sonn & Partner Patentanwälte

(57) **Abstract**

The invention discloses a pharmaceutical combination of
- a TLR3-agonist and
- Interleukin 24 (IL-24),
especially for use in the treatment of cancer or for the treatment of viral infections.

## Description

The invention relates to the field of tumor treatment and treatment of viral diseases.

The melanoma differentiation associated gene 7 (mda7) /interleukin-24 (IL-24) was identified by differentiation induction subtraction hybridization after treatment of melanoma cells with IFN-β and mezerein, a protein kinase C inhibitor, as a gene whose expression level was up-regulated in differentiated and non-proliferating melanoma cells. Shortly after its discovery, IL-24 attracted interest as a therapeutic agent since overexpression of IL-24 by a recombinant adenovirus (Ad-IL-24) induced apoptosis in cancer cells. Subsequently, the therapeutic potential of Ad-IL-24 has been confirmed in a number of different cancer models which led to further evaluation of this immunotherapy in clinical trials. Analysis of the mechanism of Ad-IL-24-induced apoptosis revealed the activation of multiple pro-apoptotic pathways including PKR phosphorylation, caspase-8 and caspase-3 cleavage and the induction of pro-apoptotic Bcl-2 family members. There is, however, conflicting data concerning the apoptosis-inducing properties of IL-24 expressed and delivered by other sources than viral overexpression. Several groups reported that IL-24 secreted from transformed embryonic kidney cells (HEK293) or bacterial GST-IL-24 fusion protein induced apoptosis in a variety of cancer cells. In contrast, other groups could not observe a reduced growth of cancer cells after treatment with IL-24

It is an object of the present invention to provide means for cancer treatment and the treatment of viral infections, especially to provide improved means based on IL-24.

Therefore, the present invention provides a pharmaceutical combination of
- a TLR3-agonist and
- Interleukin 24 (IL-24).

More specifically, the present invention provides the use of this combination in the treatment of cancer or for the treatment of viral infections. Accordingly, the present invention provides the use of a TLR3-agonist and IL-24 for the preparation of a pharmaceutical composition for the treatment of cancer or for the treatment of viral infections.

In the course of generating the present invention it was found out that the antitumor effect of IL-24 can be significantly improved by combining IL-24 with a TLR3-agonist. This combination can be used as an effective drug principle in the treatment of cancer and in the treatment of viral diseases.

The treatment of tumor diseases with the means according to the present invention is preferably performed locally (e.g. by local administration, especially by intratumoral administration (e.g. by injection)) or systemically (e.g. by intravenous or intraperitoneal administration). The cancer treatment according to the present invention is specifically suitable for solid tumors, but also for leukemias and lymphomas.

The antiviral treatment according to the present invention is preferably applied for diseases which are caused by RNA viruses (Group III, Group IV, Group V or Group VI of the Baltimore classification system of classifying viruses), such as diseases caused by coronaviridae, such as Coronavirus or SARS virus; flaviviridae, such as Yellow fever virus, West Nile virus, Hepatitis C virus, Dengue fever virus or Tick-born encephalitis virus; picornaviridae, such as Poliovirus, the common cold virus, Hepatitis A virus, paramyxoviridae, such as Measles virus, Mumps virus, Nipah virus, Hendra virus; orthomyxoviridae, such as influenza A, B or C virus; rhabdoviridae, such as Rabies virus; retroviridae, such as HIV-1 and HIV-2. The antiviral treatment with the method according to the present invention is preferably carried out by inhalation, intravenous administration or subcutaneous administration. The antiviral treatment according to the present invention is specifically suited - and therefore preferably applied for - acute infections with viruses.

IL-24 is a cytokine belonging to the IL-10 family of cytokines that signals through two heterodimeric receptors: IL-20R1/IL-20R2 and IL-22R1/IL-20R2. This interleukin gene is also known as Melanoma differentiation-associated 7 gene (mda-7) due to its discovery as a tumor suppressing protein. IL-24 appears to control in cell survival and proliferation by inducing rapid activation of particular transcription factors called Stat-1 and Stat-3. This cytokine is predominantly released by activated monocytes, macrophages and T helper 2 (Th2) cells and acts on non-haematopoietic tissues such as skin, lung and reproductive tissues. IL-24 performs important roles in wound healing, psoriasis and cancer (US 2006/0134801 A1). Several studies have shown that cell death occurs in cancer cells/cell lines following exposure to IL-24. The gene for IL-24 is located on chromosome 1 in humans. The sequence of isoform 1 of IL-24 ("canonical IL-24 sequence"; 206 amino acids; 23.8 kDa) is available under the number Q13007 (IL24_HUMAN) in the UniProtKB/Swiss-Protdatabase and in US 2006/0134801 A1.

It is contemplated that the IL-24 peptide or polypeptide employed in methods and compositions of the invention may comprise at least 10, 20, 30, 40, 50, 60, 70, 80, 90, 100, 110, 120, 130, 140, 150, 156, 157, 160, 170, 180, 190, 200 or 206 contiguous amino acids of the canonical IL-24 sequence or comprise all of the canonical IL-24 sequence. IL-24 is preferably produced by recombinant technology. The recombinant IL-24 polypeptide may be modified, or it may be truncated at either end. In some embodiments of the invention, the IL-24 polypeptide comprises amino acids 52 to 206, 75 to 206, or 100 to 206 of the canonical IL-24 sequence. The secreted form of IL-24 has amino acids 52 to 206 of the canonical IL-24 sequence, but the first 51 amino acids are absent, and it is specifically contemplated that the secreted form qualifies as "the IL-24 polypeptide" and may be used in any composition or method of the invention. Alternatively, an IL-24 amino acid sequence may include a heterologous amino acid sequence, such as a secretory signal. In some embodiments, the secretory signal is a positively charged N-terminal region that has a hydrophobic core. In other embodiments, the secretory signal targets IL-24, or a truncation thereof, to the endoplasmic reticulum or mitochondria.

Expression constructs for recombinantly producing IL-24 may be viral or nonviral vectors. Viral vectors that are considered part of the invention include, but are not limited to, adenovirus, adeno-associated virus, herpesvirus, retrovirus (including lentiviruses), polyoma virus, influenza virus, or vacciniavirus, however, expression vectors which are members of the RNA virus family are specifically preferred, because these systems can also be used for eliciting TLR3-stimulation. Accordingly, such expression systems for IL-24 are not only suitable for the production of (recombinant) IL-24, but can be used as a combination agent according to the present invention. In the case a RNA virus vector is used as a combination agent according to the present invention, it is preferred to provide the IL-24 gene with the 51 amino acid signal sequence.

Besides human IL-24, which is the most preferred ortholog to be used in the present invention, because the central patient in the treatments according to the present invention is the human patient, other orthologs are known e.g. in chimpanzee (Pan troglodytes) (available under the numbers 457683, XM_5l4l5l.2, XP_514151.2 via the GeneCards library), mouse (Mus musculus) (GeneCard: 936721, NM_053095.11, NP_444325.11, AF2350065 AF3332515), rat (Rattus norvegicus) (GeneCard: 170819, NM_133311.1, NP_579845.1), cow (Bos taurus) (GeneCard: 526285, XM_604649.3, XP_604649.3), dog (Canis familiaris) (GeneCard: 609213, XM_846427.1, XP_851520.1), opossum (Monodelphis domestica) (GeneCard: 2(113371072-113377464), platypus (Ornithorhynchus anatinus) ("interleukin 19"; GeneCard: Contig3915 (14622-22601), lizard (Anolis carolinensis) (GeneCard: 1(1949292-1953668)), and zebrafish (Danio rerio) ("interleukin 34"; GeneCard: 11(21929589-2193).

However, as already stated above, the pharmaceutical combination according to the present invention preferably comprises as IL-24 a human recombinant IL-24.

According to the present invention, IL-24 is combined with a TLR3-agonist. TLR3 is a member of the Toll-like receptor (TLR) family which plays a fundamental role in pathogen recognition and activation of innate immunity. TLRs are highly conserved from Drosophila to humans and share structural and functional similarities. They recognize pathogen-associated molecular patterns (PAMPs) that are expressed on infectious agents, and mediate the production of cytokines necessary for the development of effective immunity. The various TLRs exhibit different patterns of expression. This receptor is most abundantly expressed in placenta and pancreas, and is restricted to the dendritic subpopulation of the leukocytes. It recognizes dsRNA associated with viral infection, and induces the activation of NF-κB and the production of type I interferons. It may thus play a role in host defence against viruses.

TLR3 recognizes double-stranded RNA, a form of genetic information carried by some viruses such as retroviruses. Upon recognition, TLR 3 induces the activation of NF-κB to increase production of type I interferons which signal other cells to increase their antiviral defences.

TLR3-agonists are well known in the art, also in the use for treatment of cancer, e.g. from US 2008/0253998 A1, WO 2009/130301 A1 and US 2012/0045523 A1). The TLR3-agonists according to the present invention can be selected from any suitable agent. For example, TLR3-agonists can be selected from a range of nucleic acid agonists; other agonists, whether nucleic acid based, proteinaceous or small molecules, can be tested using known assays. Generally, any proteinaceous, nucleic acid or small molecule candidate TLR3-agonist can be identified using known assays. For example, assays for detecting TLR3 agonism of test compounds are described, for example, (summarised and incorporated by reference in US 2008/0253998 A1, [0099]). Regardless of the particular assay employed, a compound can be identified as an agonist of TLR3 if performing the assay with a compound, which results in at least a threshold increase of some biological activity mediated by TLR3. Conversely, a compound may be identified as not acting as an agonist of TLR3 if, when used to perform an assay designed to detect biological activity mediated by TLR3, the compound fails to elicit a threshold increase in the biological activity. Unless otherwise indicated, an increase in biological activity refers to an increase in the same biological activity over that observed in an appropriate control. An assay may or may not be performed in conjunction with the appropriate control. With experience, one skilled in the art may develop sufficient familiarity with a particular assay (e.g., the range of values observed in an appropriate control under specific assay conditions) that performing a control may not always be necessary to determine the TLR3 agonism of a compound in a particular assay. The precise threshold increase of TLR3-mediated biological activity for determining whether a particular compound is or is not an agonist of TLR3 in a given assay may vary according to factors known in the art including but not limited to the biological activity observed as the endpoint of the assay, the method used to measure or detect the endpoint of the assay, the signal-to-noise ratio of the assay, the precision of the assay. For example, regardless of the particular assay employed, a compound can generally be identified as an agonist of TLR3 if performing the assay with a compound results in at least a threshold increase of some biological activity mediated by TLR3. Assays employing HEK293 cells transfected with an expressible TLR3 structural gene may use a threshold of, for example, at least a three-fold increase in a TLR3-mediated biological activity (e.g., NF-KB activation) when the compound is provided at a concentration of, for example, from about 1 µM to about 10 µM for identifying a compound as an agonist of the TLR3 transfected into the cell. However, different thresholds and/or different concentration ranges may be suitable in certain circumstances. Also, different thresholds may be appropriate for different assays.

In certain embodiments, the TLR3-agonist can be a natural agonist of a TLR3 or a synthetic TLR3-agonist compound. In preferred embodiments of the invention, a TLR3-agonist is used to treat a patient. TLR3-agonists are well known in the art and suitable TLR3-agonists are available. Further TLR3-agonists, or derivatives or analogs of known TLR3-agonists can be readily identified, made and/or assessed.

The most commonly used TLR3-agonistare nucleic acid based agonists. Thus in preferred aspects, a TLR3-agonist for use according to the present invention are nucleotide or nucleic acid based. Nucleotide or nucleic-acid based compounds can be assessed for their ability to act as a TLR3-agonist using readily available methods. The nucleic acid based TLR3-agonist can be single-stranded or double-stranded or a mixture thereof. The nucleic acid based TLR3-agonist can comprise deoxyribonucleotides, or ribonucleotides or a mixture thereof. The nucleotides can be natural or synthetic, and may be derivatives or analogs of natural nucleotides.

The preferred group of TLR-agonists according to the present invention is double stranded RNA compound (dsRNA), especially polyadenylic-polyuridylic acid, i.e., poly (A): poly (U), polyAU or poly A: U. Double-stranded RNA which represents either genomic or life cycle intermediate material of many viruses activates cells through binding to the dsRNA-dependent protein kinase (PKR), a kinase that initiates a complex molecular anti-viral program. Induction of apoptosis by double-stranded-RNA-dependent protein kinase (PKR) involves the alpha subunit of eukaryotic translation initiation factor 2 and NF-kappaB. It was reported that dsRNA triggers the production of type 1 IFN, and dsRNA has been reported to have promise for certain clinical applications such as anti-viral therapies. A dsRNA compound referred to as Ampligen, for example, has been studied for its ability induce type 1 IFN production and as a consequence to treat viral infection.

Within the context of the present invention, the term "double-stranded RNA" molecule designates any therapeutically or prophylactically effective (synthetic) double-stranded RNA compound. Such compounds are typically active per se, i.e., they do not encode a polypeptide or do not require translation to be active. dsRNATLR3-agonists can have any suitable length. Preferably, a dsRNA molecule TLR3-agonist has a length of at least about 10 base pairs (bp), 20 bp, 30 bp, 50 bp, 80 bp, 100 bp, 200 bp, 400 bp, 600 bp, 800 bp or 1000 bp. In one aspect the dsRNA molecule is a short dsRNA having a chain length of less than 30 bp, 50 bp, 80 bp, 100 bp or 200 bp. In another embodiment, the dsRNA molecule is a longer dsRNA, but having a chain length of less than 400 bp, 600 bp, 800 bp or 1000 bp. In another embodiment, the dsRNA molecule is a long dsRNA having a chain length of greater than 1000 bp. In one aspect, a dsRNA composition comprises a heterogeneous mixture of dsRNA molecules, wherein a plurality of molecules has differing lengths. Preferably the dsRNA molecules have on average a length of at least about 10 bp, 20 bp, 30 bp, 50 bp, 80 bp, 100 bp, 200 bp, 400 bp, 600 bp, 800 bp or 1000 bp. In another embodiment, a dsRNA composition comprises a plurality dsRNA molecules where at least 20%, 50%, 80%, 90% or 98% of dsRNA molecules have a length of at least about 10 bp, 20 bp, 30 bp, 50 bp, 80 bp, 100 bp, 200 bp, 400 bp, 600 bp, 800 bp or 1000 bp. In a preferred embodiment dsRNA composition has a substantially homogenous mixture of dsRNA molecules, where substantially all the molecules do not differ in chain length by more than 30 bp, 50 bp, 80 bp, 100 bp or 200 bp. Average chain length of nucleic acid TLR3-agonists can be determined easily, for example, by gel permeation chromatography.

Previous studies of double-stranded RNA (dsRNA) assessing their ability to be effective interferon inducers suggested that dsRNA agents must possess the secondary structure of a double stranded helix. Other dsRNA agents which have also been shown to be suitable as TLR3-agonist include double-stranded polynucleotides which are not complementary or not perfectly complementary; these have been known as, so-called "mismatched" or "loop-out" structures and exist in naturally occurring RNAs such as transfer tRNAs, ribosomal RNAs and the viral RNA secondary structures. One commonly cited dsRNA compound, Ampligen, comprises a structure where a few parts of cytidine in the poly I: poly C structure are replaced with uridine (i.e. mismatched RNA); this compound has been reported to have physiological activity similar to that of the parent poly I: poly C. However it will be appreciated that TLR3-agonists of any type and configuration can be used in accordance with this invention.

Generally, the polynucleotides need to be resistant to nucleases in order to remain as macromolecules for a sufficient length of time; polynucleotides are less sensitive to nuclease attack when they are in a helical complex. However, certain analogs such as Ampligen(TM) appear to retain their TLR3-agonist activity.

In a particular embodiment, each strand of these dsRNAs can have a length comprised between about 5 and 50 bases, more preferably between 5 and 40, 35, 30, 25 or 20 bases. Each strand is preferably perfectly complementary to the other. Preferred examples of such dsRNAs are homopolyRNAs, i.e., dsRNAs in which each strand comprises essentially a repeat of the same base; or comprise a homopolyRNA region.

The base may be any naturally occurring base (e.g., polyA, polyU, polyC, polyG) or non-naturally occurring (e.g., chemically synthesized or modified) base (e.g., polyI). Polynucleotides typified by polyinosinic-polycytidylic acid, i.e., poly (I): poly(C) or poly I: C and polyadenylic-polyuridylic acid, i.e., poly (A): poly (U) or poly A: U, are well-known compounds in the art and have been known to induce interferon production by immune cells. Thus in preferred embodiments, the TLR3-agonist for use according to the invention is a double stranded nucleic acid selected from the group consisting of: polyinosinic acid and polycytidylic acid, polyadenylic acid and polyuridylic acid, polyinosinic acid analogue and polycytidylic acid, polyinosinic acid and polycytidylic acid analogue, polyinosinic acid analogue and polycytidylic acid analogue, polyadenylic acid analogue and polyuridylic acid, polyadenylic acid and polyuridylic acid analogue, and polyadenylic acid analogue and polyuridylic acid analogue.

It will be appreciated that nucleic acid-based agonists of TLR3 can be designed using any suitable method. Preferably, the basic requirement of stability and resistance to nuclease attack and the preferences for chain length are taken into account, and that structural changes can be tested and assessed with reference to the a rAₙ: rUₙ or rIₙ: rCₙ complex for example. Measures can be taken to increase stability and resistance to nucleases, or to increase or optionally decrease interferon-inducing action.

It was disclosed that when chain length of the double stranded nucleic acid derivatives is limited to certain ranges, the resulting substances exhibit desired physiological activity with markedly less toxicity, providing polynucleotides having a length of about 50 to 10,000 as calculated by base pair numbers. Also described are derivative wherein the purine or pyrimidine ring in the nucleic acid polymer is substituted with at least one SH group, or said derivative contains a disulphide bond, or both (preferred ratio of number of sulphur atoms to cytidylic acid present in the poly C are 1:6 to 39). Also a particular type of dsRNA compounds is disclosed that are "chain-shortened" having lengths of about 100 to 1,000 as calculated by base pair numbers, or preferably from 200 to 800, and more preferably from 300 to 600. The latter compounds are reported to contain low numbers of 2'-5' phosphodiester bonds by a method designed to avoid phosphate groups causing intramolecular rearrangement from 3' position to 2' position through a mechanism called pseudo rotation simultaneously that can occur during hydrolysis of polynucleotides, resulting in a portion of 3'-5' phosphodiester bonds in the chain-shortened polynucleotide molecule being replaced by 2'-5' phosphodiester bonds. A number of synthetic nucleic acid derivatives have been described, including homopolymer-homopolymer complexes (Double Strand Nucleic Acid Polymer such as those in which poly I:C or poly A:U are a parent structure, where these homopolymer-homopolymer complexes contain: (1) base modifications, exemplified by Polyinosinic acid-poly(5-bromocytidylic acid), Polyinosinic acid-poly(2-thiocytidylic acid), Poly(7-deazainosinic acid)-polycytidylic acid, Poly(7-deazainosinic acid)-poly(5-bromocytidylic acid), and Polyinosinic acid-poly(5-thiouridylic acid); (2) Sugar Modifications, exemplified by Poly(2'-azidoinosinic acid)-polycytidylic acid; and (3) Phosphoric Acid Modifications, exemplified by Polyinosinic acid-poly(cytidyl-5'-thiophosphoric acid). Other synthetic nucleic acid derivatives that have been described include interchanged copolymers, exemplified by Poly (adenylic acid-uridylic acid); and homopolymer-copolymer complexes, exemplified by Polyinosinic acid-poly (cytidylic acid-uridylic acid) and Polyinosinic acid-poly (citydylic acid-4-thiouridylic acid). Other synthetic nucleic acid derivatives that have been described include complexes of synthetic nucleic acid with polycation, exemplified by Polyinosinic acid-polycytidylic acid-poly-L-lysinecarboxy-methylcellulose complex (called "Poly ICLC"). Yet another example of synthetic nucleic acid derivative is Polyinosinic acid-poly (1-vinylcytosine). One example of a TLR3-agonist is Ampligen(TM) (Hemispherx, Inc., of Rockville, Md., U.S.A.), a dsRNA formed by complexes of polyriboinosinic and polyribocytidylic/uridylic acid, such as rIₙ: r(Cₓ, U or G)n where x has a value from 4 to 29, e.g., rIₙ: r (C₁₂ U)ₙ. Many mismatched dsRNA polymers which behave similarly to Ampligen have been studied; mismatched dsRNA based on poly I: C has included complexes of a polyinosinate and a polycytidylate containing a proportion of uracil bases or guanidine bases, e.g., from 1 in 5 to 1 in 30 such bases. A key therapeutic advantage of mismatched dsRNAs over other forms of natural and/or synthetic dsRNAsis a reduction in toxicity.

Specific examples of double-stranded RNA according to the present invention further include Polyadenur (Ipsen) and Ampligen (Hemispherx). Polyadenur is a polyA/U RNA molecule, i.e., contains a polyA strand and a polyU strand. Polyadenur has been developed for the potential treatment of hepatitis B virus (HBV) infection. Ampligen is of a polyI/polyC compound (or a variant thereof comprising a polyI/polyC12U RNA molecule). Ampligen has been proposed for the treatment of cancer, viral infections and immune disorders. It was developed primarily for the potential treatment of myalgic encephalomyelitis (ME, or chronic fatigue syndrome/chronic fatigue immune dysfunction syndrome, CFS/CFIDS).

A particular example of a dsRNA for use in the present invention is a dsRNA comprising a polyA/polyU region, wherein each strand of said dsRNA contains less than 25 bases. Another particular example of a dsRNA for use in the present invention is a dsRNA comprising a polyI/polyC (U) region, wherein each strand of said dsRNA contains less than 25 bases. Further dsRNAs have been disclosed in the literature or may be developed, which can be used within the present invention (for a summary: see US 2008/0253998 A1). More generally, any synthetic double-stranded homopolyRNA may be used in the context of this invention.

TLR3-agonist can also be any organic or inorganic substance, such as a lipid, peptide, polypeptide, small molecule, etc., in isolated or in mixture with other substances. The TLR3-agonist candidate may be selected from a combinatorial library of products, for instance. In a preferred embodiment, the TLR3-agonist is an antibody directed against TLR3 receptor and which is capable of activating a TLR3 receptor to induce a full or partial receptor-mediated response. The TLR3-agonist can also be an antibody fragment or derivative of an antibody directed against TLR3 receptor and which is capable of activating a TLR3 receptor to induce a full or partial receptor-mediated response.

Accordingly, the pharmaceutical combination according to the present invention preferably contains a double-stranded RNA (dsRNA) as the TLR3-agonist especially poly(A:U)-dsRNA and poly(I:C)-dsRNA.

In a preferred embodiment of the present invention, the pharmaceutical combination is present in a single pharmaceutical preparation.

However, at least for reasons of storage, it may also be preferred to provide the pharmaceutical combination according to the present invention in separated form, i.e. wherein the TLR3-agonist and the IL-24 are provided in separate pharmaceutical preparations.

The pharmaceutical combination according to the present invention is preferably applied in a form wherein the IL-24 and/or the TLR3-agonist further comprise a pharmaceutically acceptable excipient. The phrase "pharmaceutically acceptable" refers to molecular entities and compositions that do not produce an allergic or similar untoward reaction when administered to a human. The preparation of an aqueous composition that contains a protein or a nucleic acid as an active ingredient (or a combination thereof) is well understood in the art. Typically, such compositions are prepared as injectables, either as liquid solutions or suspensions; solid forms suitable for solution in, or suspension in, liquid prior to injection can also be prepared. Typically, adjuvants can be provided in order to enhance the immunogenicity of a nucleic acid or IL-24. Adjuvants are usually used as non-specific stimulators of the immune response. Suitable adjuvants include all acceptable immunostimulatory compounds, especially a molecule that increases expression of PKR. Suitably adjuvants may be BCG, aluminum hydroxide, MDP compounds, such as thur-MDP and nor-MDP, CGP (MTP-PE), lipid A, and monophosphoryl lipid A (MPL), Freund's adjuvant (a non-specific stimulator of the immune response containing killed Mycobacterium tuberculosis), incomplete Freund's adjuvants and aluminum hydroxide adjuvant.

In certain specific embodiments, it is desired to kill cells, inhibit cell growth, inhibit metastasis, decrease tumor or tissue size and otherwise reverse or reduce the malignant phenotype of tumor cells, induce an immune response, or inhibit angiogenesis using the methods and compositions of the present invention. The routes of administration will vary, naturally, with the location and nature of the lesion or site to be targeted, and include, e.g., intradermal, subcutaneous, regional, parenteral, intravenous, intramuscular, intranasal, systemic, and oral administration and formulation. However, especially due to the nature of IL-24, direct injection, intratumoral injection, or injection into the tumor vasculature is specifically contemplated for discrete, solid, accessible tumors or other accessible target areas. Local or regional administration also may be appropriate. For tumors of >4 cm, the volume to be administered will be about 4-10 ml (preferably 10 ml), while for tumors of <4 cm, a volume of about 1-3 ml will be used (preferably 3 ml). Multiple injections delivered as single dose comprise about 0.1 to about 0.5 ml volumes. The viral particles may advantageously be contacted by administering multiple injections to the tumor or targeted site, spaced at approximately 1 cm intervals. In the case of surgical intervention, the present invention may be used preoperatively, to render an inoperable tumor subject to resection. Alternatively, the present invention may be used at the time of surgery, and/or thereafter, to treat residual or metastatic disease. For example, a resected tumor bed may be injected or perfused with a formulation comprising MDA-7 or an MDA-7-encoding construct together with or in the absence of an immunogenic molecule. The perfusion may be continued post-resection, for example, by leaving a catheter implanted at the site of the surgery. Periodic post-surgical treatment also is envisioned. Continuous perfusion of an expression construct or a viral construct also is contemplated. The amount of construct or peptide delivered in continuous perfusion can be determined by the amount of uptake that is desirable. Continuous administration also may be applied where appropriate, for example, where a tumor or other undesired affected area is excised and the tumor bed or targeted site is treated to eliminate residual, microscopic disease. Delivery via syringe or catherization are examples for continuous administration. Such continuous perfusion may take place for a period from about 1-2 hours, to about 2-6 hours, to about 6-12 hours, to about 12-24 hours, to about 1-2 days, to about 1-2 wk or longer following the initiation of treatment. Generally, the dose of the therapeutic composition via continuous perfusion will be equivalent to that given by a single or multiple injections, adjusted over a period of time during which the perfusion occurs.

Treatment regimens may vary as well, and often depend on tumor type, tumor location, immune condition, target site, disease progression, and health and age of the patient. Obviously, certain types of tumors will require more aggressive treatment, while at the same time, certain patients cannot tolerate more taxing protocols. The clinician will be best suited to make such decisions based on the known efficacy and toxicity (if any) of the therapeutic formulations. In certain embodiments, the tumor or affected area being treated may not, at least initially, be resectable. Treatments with therapeutic viral constructs may increase the resectability of the tumor due to shrinkage at the margins or by elimination of certain particularly invasive portions. Following treatments, resection may be possible. Additional treatments subsequent to resection will serve to eliminate microscopic residual disease at the tumor or targeted site. A typical course of treatment, for a primary tumor or a post-excision tumor bed, will involve multiple doses. Typical primary tumor treatment involves a 6 dose application over a two-week period. The two-week regimen may be repeated one, two, three, four, five, six or more times. During a course of treatment, the need to complete the planned dosings may be re-evaluated. The treatments may include various "unit doses." Unit dose is defined as containing a predetermined-quantity of the therapeutic composition (i.e. the pharmaceutical combination or preparations disclosed according to the present invention). The quantity to be administered, and the particular route and formulation, are within the skill of those in the clinical arts. A unit dose need not be administered as a single injection but may comprise continuous infusion over a set period of time.

IL-24 may be administered to a patient in doses of or of at least 0.01. 0.05, 0.1, 0.2, 0.3, 0.4, 0.5, 0.6, 0.7, 0.8, 0.9, 1.0, 2.0, 3.0, 4.0, 5.0, 6.0, 7.0, 8.0. 9.0, 10, 15, 20, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950, 1000, 2000, 3000, 4000, 5000, 6000, 7000, 8000, 9000, 10000 or more ng/ml.

The TLR3-agonist (preferably, a dsRNA) is preferably administered to a subject using a dosing regimen that maintains the plasma concentration of the agonist at a desirable level. In a specific embodiment, the plasma concentration of the dsRNA is maintained at 10 µg/ml, 15 µg/ml, 20 µg/ml, 25 µg/ml, 30 µg/ml, 35 µg/ml, 40 µg/ml, 45 µg/ml or 50 µg/ml. The plasma concentration that is desirable in a subject will vary depending on several factors including, but not limited to, the nature of the cancer, the severity of the cancer, and the circulation half-life (stability) and binding affinity of the TLR3-agonist.

Since it is usually desirable in tumor treatment to provide the combination according to the present invention close to the tumor and have a steady concentration of the antitumor agents there, it is preferred to provide the combination according to the present invention as an implantable drug release device, especially a drug release device which allows slow release.

According to a specific embodiment of the present invention, the combination according to the present can be provided in the form of an IL-24 expressing viral vector which itself produces a TLR3 signal. Therefore, the present invention also contemplates a pharmaceutical preparation comprising a viral vector being a TLR3-agonist and being capable of expressing IL-24.

This pharmaceutical preparation comprising a viral vector being a TLR3-agonist and being capable of expressing IL-24 is specifically suited for use in the treatment of cancer or for the treatment of a patient suffering from a viral infection. The TLR3-agonist can therefore be a specific type of molecule (e.g. an RNA molecule) or a (viral) vector. According to a preferred embodiment, the viral vector is an influenza virus vector, preferably an influenza A virus vector. A specifically preferred embodiment according to the present invention is an influenza A virus wherein the codon for amino acid no. 20 of the nuclear export protein (NEP) gene, glutamine, has been changed to encode for arginine, histidine, lysine, asparagine, aspartic acid, or glutamic acid, especially arginine. Even more preferred, such influenza A viruses are constructed as expression systems for IL-24, i.e. further comprising a IL-24 gene.

A preferred embodiment of the pharmaceutical combination according to the present invention comprises viral vectors which stimulate TLR3 or RNA molecules.

According to another aspect, the present invention relates to a method for treating a cancer patient or for the treatment of a patient suffering from a viral infection comprising administering an effective amount of a pharmaceutical combination or a pharmaceutical preparation according to the present invention.

Preferably, the administering is performed by administering a single dosage to the cancer patient or the patient suffering from a viral infection. Single (or unit) dose is defined as containing a predetermined-quantity of the therapeutic composition. The quantity to be administered, and the particular route and formulation, are within the skill of those in the clinical arts. A single or unit dose need not be administered as a single injection but may comprise continuous infusion over a set period of time

Preferred administration regimen includes dosage forms, wherein the single dosage of the TLR3-agonist and IL-24 is (especially if applied subcutaneously, intraperitoneally, intratumorally or intravenously), independently of each other, is 0.1 to 100 mg/kg body weight, preferably 0.5 to 50 mg/kg body weight, especially 1 to 10 mg/kg body weight, of the TLR3-agonist, and 0.01 to 10000 ng/ml, preferably 1 to 1000 ng/ml, especially 30 to 300 ng/ml, of IL-24. If the TLR3-agonist is applied as viral vector, specifically suitable administration dosages are 10⁴ to 10¹⁰ pfu/ml, preferably 10⁵ to 10⁹ pfu/ml, especially 10⁶ to 10⁸ pfu/ml, of the TLR3-agonist.

A specifically preferred embodiment of the present invention is a method wherein repetitive administration such as intratumoral, intravenous or inhalation (antiviral).

The invention is further illustrated by the following examples and the figures, yet without being restricted thereto.

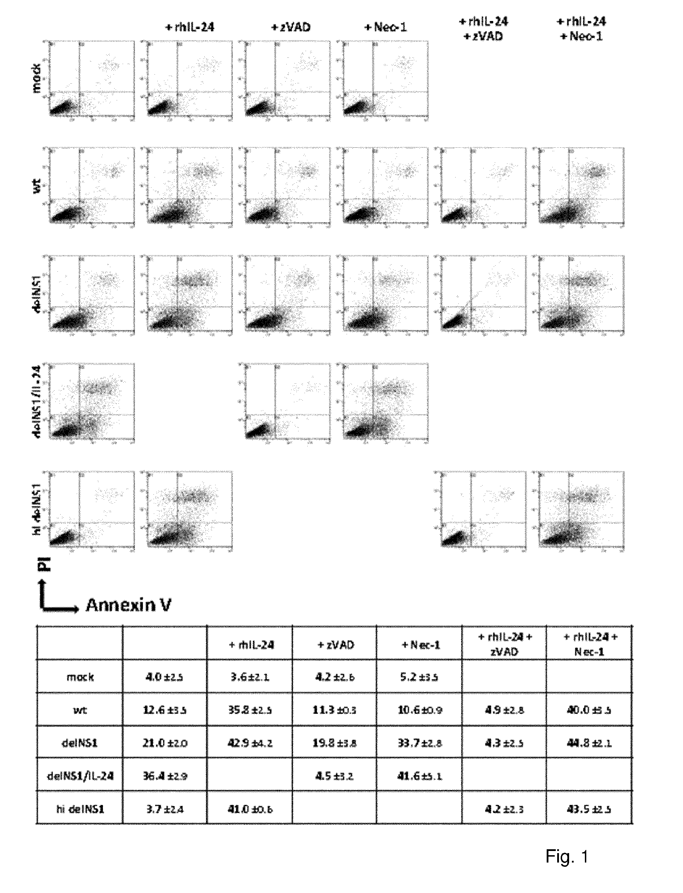

Figure 1 shows the effect of IL-24 and/or virus stimulation on apoptosis in DU145 cells. Apoptosis was determined by AnnexinV/PI stainings of cells treated for 24 hours. Cells were infected with wild-type (wt), delNS1, delNS1/IL-24, heat inactivated (hi) delNS1 (moi=1) indicated on the left or in combination with recombinant human (rh) IL-24 (100ng/ml), zVAD (10µM) and Necrostatin-1 (Nec-1) (50µM) indicated on the top. The lower panel shows the percentage of early and late apoptotic cells (mean from three independent experiments with SEM).

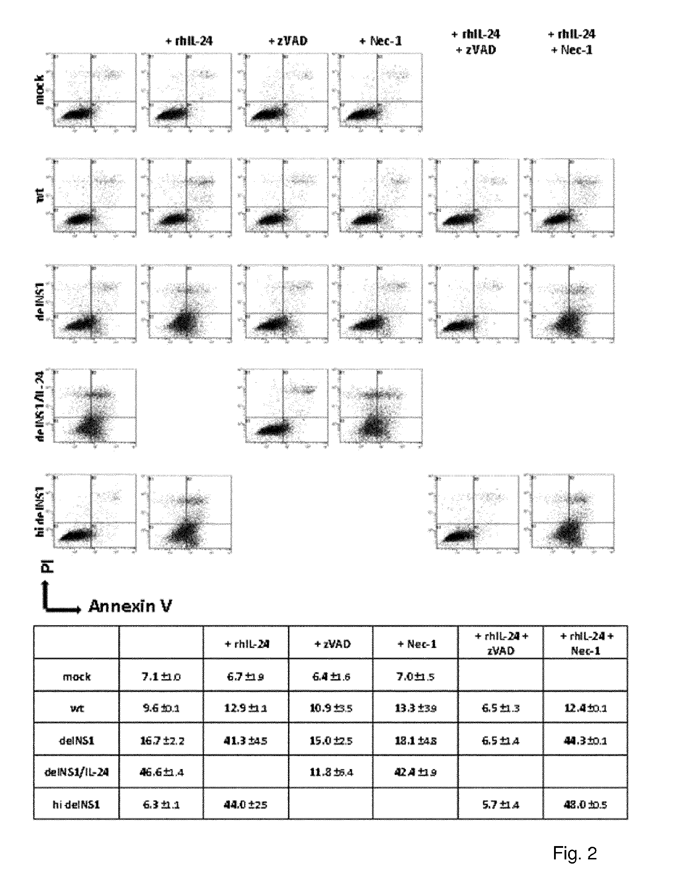

Figure 2 shows the effect of IL-24 and/or virus stimulation on apoptosis in SK-Mel28 cells. Apoptosis was determined by AnnexinV/PI stainings of cells treated for 24 hours. Cells were infected with wild-type (wt), delNS1, delNS1/IL-24, heat inactivated (hi) delNS1 (moi=1) indicated on the left or in combination with recombinant human (rh) IL-24 (100ng/ml), zVAD (10µM) and Necrostatin-1 (Nec-1) (50µM) indicated on the top. The lower panel shows the percentage of early and late apoptotic cells (mean from three independent experiments with SEM).

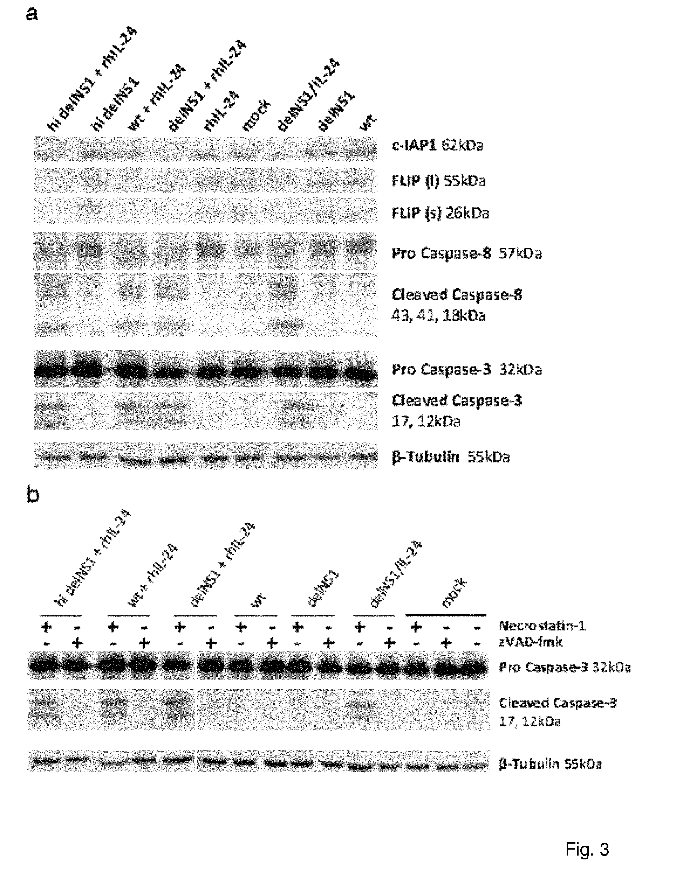

Figure 3 shows the effect of IL-24 and/or virus stimulation on apoptosis involved proteins cIAP1, cFLIP, caspase-8 and caspase-3 in DU145 cells.(a) Cells were infected with wild-type (wt), delNS1, delNS1/IL-24, heat inactivated (hi) delNS1 (moi=1) or in combination with recombinant human IL-24 (100ng/ml) for 24 hours, indicated on the top. Protein expression level of cIAP1, cFLIP_{L}, cFLIP_{S}, caspase-8 and caspase-3 was determined by western blot analysis, indicated on the right. One representative image (out of three experiments) is shown.(b) DU145 cells were infected with wt, delNS1, delNS1/IL-24 and hi delNS1 (moi=1) alone or in combination with rhIL-24 (100ng/ml), indicated on the top. Experiments shown were performed in the presence of 50µM Necrostatin-1 or 10µM zVAD, indicated on the right. One representative western blot (out of three experiments) of caspase-3 cleavage is shown.

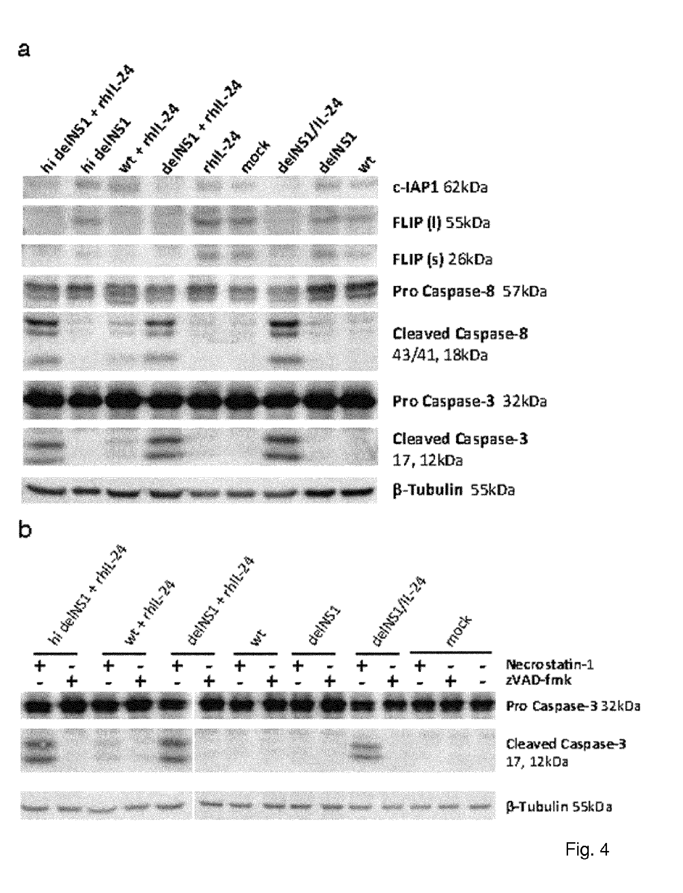

Figure 4 shows the effect of IL-24 and/or virus stimulation on apoptosis involved proteins cIAP1, cFLIP, caspase-8 and caspase-3 in SK-Mel28 cells.(a) Cells were infected with wild-type (wt), delNS1, delNS1/IL-24, heat inactivated (hi) delNS1 (moi=1) or in combination with recombinant human IL-24 (100ng/ml) for 24 hours, indicated on the top. Protein expression level of cIAP1, cFLIP_{L}, cFLIP_{S}, caspase-8 and caspase-3 was determined by western blot analysis, indicated on the right. One representative image (out of three experiments) is shown.(b) Cells were infected with wt, delNS1, delNS1/IL-24 and hi delNS1 (moi=1) alone or in combination with rhIL-24 (100ng/ml), indicated on the top. Experiments shown were performed in the presence of 50µM Necrostatin-1 or 10µM zVAD, indicated on the right. One representative western blot (out of three experiments) of caspase-3 cleavage is shown.

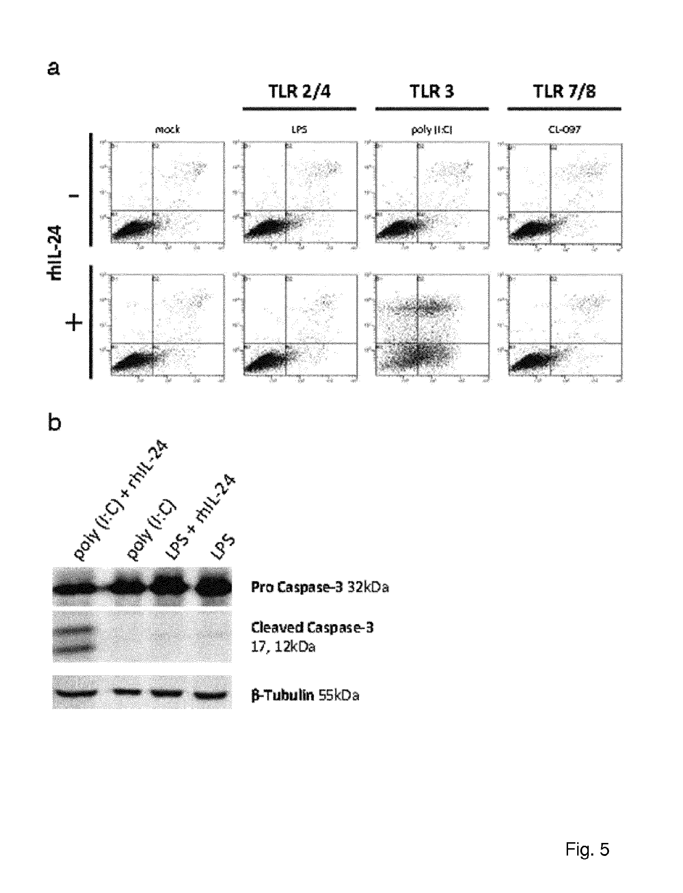

Figure 5 shows the influence of TLR-3 signaling in connection with recombinant human IL-24 in DU145 cells. (a) Cells were treated with LPS (100ng/ml), poly (I:C) (2µg/ml) and CL-097 (2,5µg/ml) for 24 hours alone or in combination with rhIL-24 (100ng/ml). Shown is one representative Annexin V/PI double staining (out of three experiments), followed by flow cytometry analysis. (b) Cells were treated for 24 hours with LPS and poly (I:C) alone or in combination with rhIL-24. One representative western blot (out of three experiments) of caspase-3 cleavage is shown.

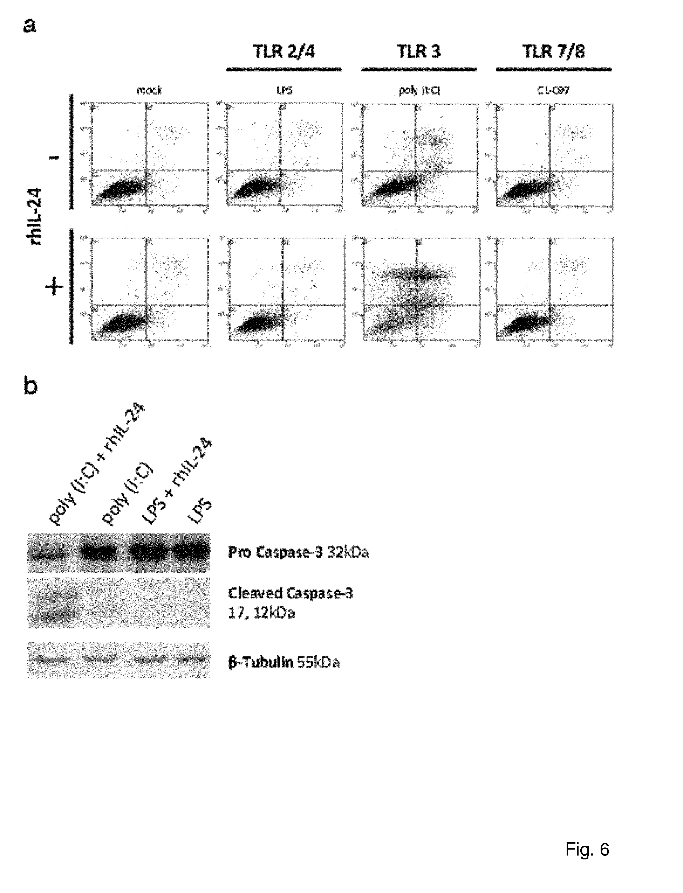

Figure 6 shows the influence of TLR-3 signaling in connection with recombinant human IL-24 in SK-Mel28 cells. (a) Cells were treated with LPS (100ng/ml), poly (I:C) (2µg/ml) and CL-097 (2,5µg/ml) for 24 hours alone or in combination with rhIL-24 (100ng/ml). Shown is one representative Annexin V/PI double staining (out of three experiments), followed by flow cytometry analysis. (b) Cells were treated for 24 hours with LPS and poly (I:C) alone or in combination with rhIL-24. One representative western blot (out of three experiments) of caspase-3 cleavage is shown.

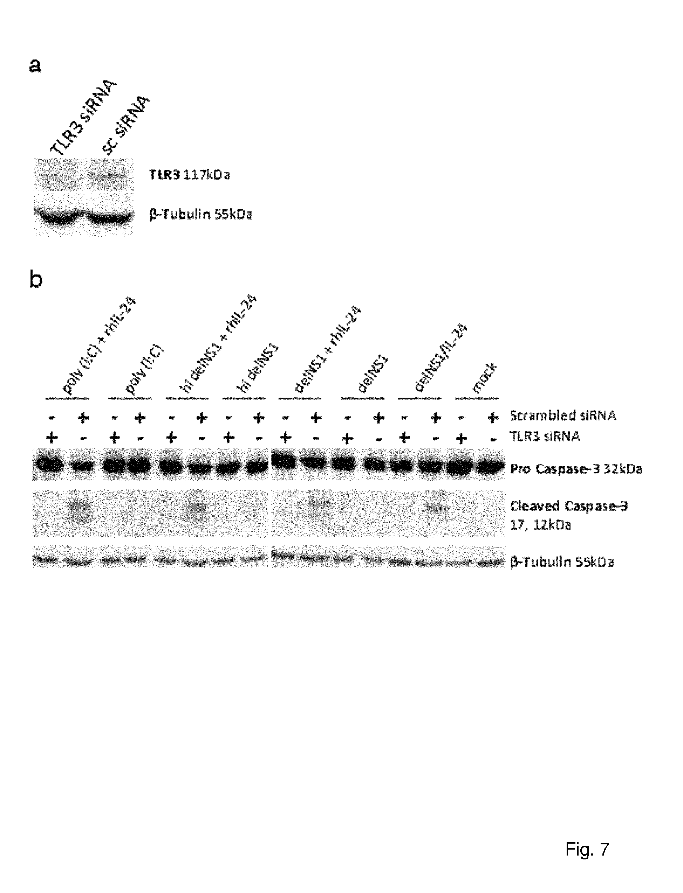

Figure 7 shows the effect of TLR-3 silencing on DU145 cells after IL-24 and /or virus stimulation.(a) TLR3 siRNA is effectively reducing TLR-3 expression as determined by western blot analysis of the TLR-3 protein.
   (b) Scrambled or TLR-3 siRNA transfected DU145 cells were infected for 24 hours with wild-type (wt), delNS1, delNS1/IL-24, heat inactivated (hi) delNS1 (moi=1), poly (I:C) (2µg/ml) alone or in combination with recombinant human (rh) IL-24 (100ng/ml). Apoptosis was demonstrated by cleavage of caspase-3 using western blot analysis. One representative image (out of three experiments) is shown.

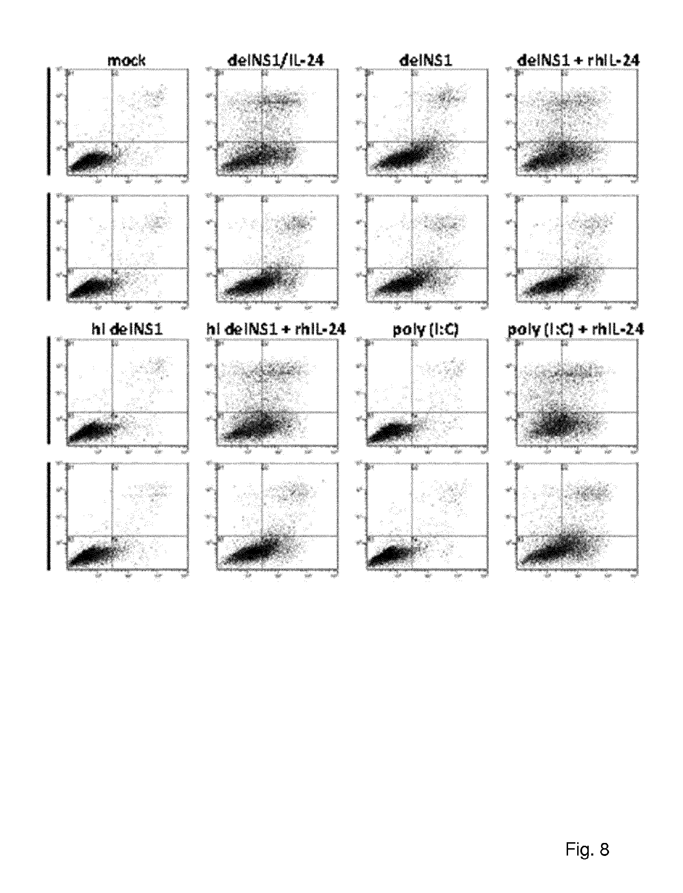

Figure 8 shows the effect of Resveratrol and IL-24 and/or virus stimulation on DU145 cells.DU145 cells were treated with 50µM Resveratrol after single infection of delNS1, delNS1/IL-24, heat inactivated delNS1 (moi=1) and poly (I:C)(2µg/ml) alone or in combination with recombinant human IL-24 (100ng/ml) for 24 hours. Shown is one representative Annexin V/PI double staining (out of three experiments) followed by flow cytometryanalysis.

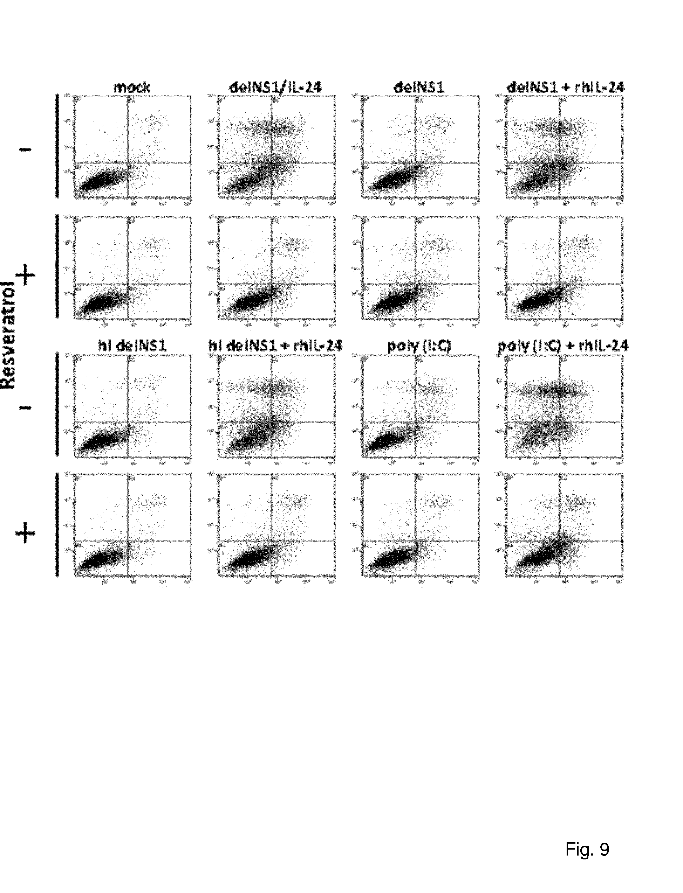

Figure 9 shows the effect of Resveratrol and IL-24 and/or virus stimulation on SK-Mel28 cells. Cells were treated with 50µM Resveratrol after single infection of delNS1, delNS1/IL-24, heat inactivated delNS1 (moi=1) and poly (I:C)(2µg/ml) alone or in combination with recombinant human IL-24 (100ng/ml) for 24 hours. Shown is one representative Annexin V/PI double staining (out of three experiments) followed by flow cytometryanalysis.

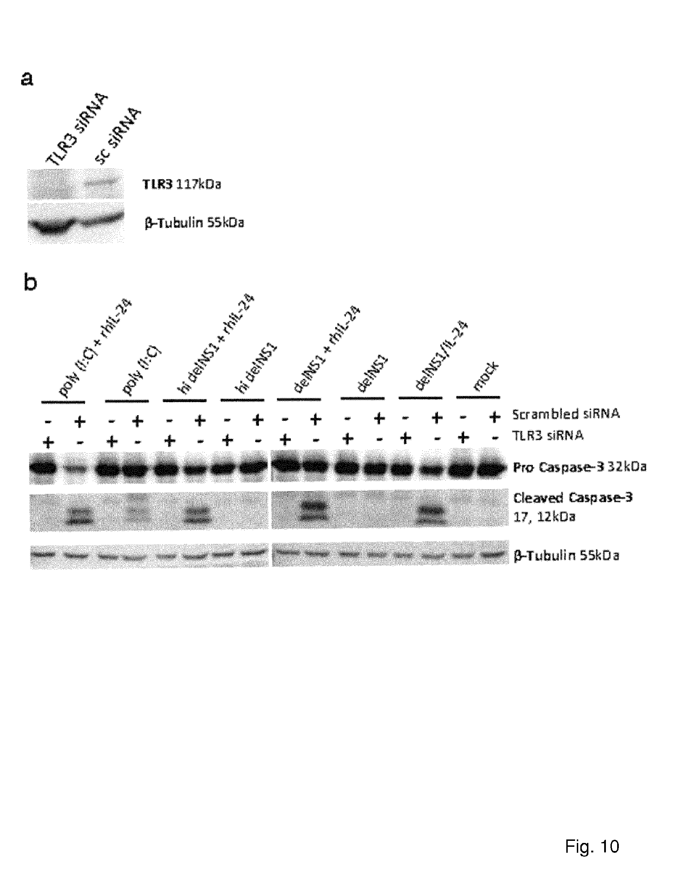

Figure 10 shows the effect of TLR-3 silencing on SK-Mel28 cells after IL-24 and /or virus stimulation.(a) TLR3 siRNA is effectively reducing TLR-3 expression as determined by western blot analysis of the TLR-3 protein. (b) Scrambled or TLR-3 siRNAtransfected DU145 cells were infected for 24 hours with wild-type (wt), delNS1, delNS1/IL-24, heat inactivated (hi) delNS1 (moi=1), poly (I:C) (2µg/ml) alone or in combination with recombinant human (rh) IL-24 (100ng/ml). Apoptosis was demonstrated by cleavage of caspase-3 using western blot analysis. One representative image (out of three experiments) is shown.

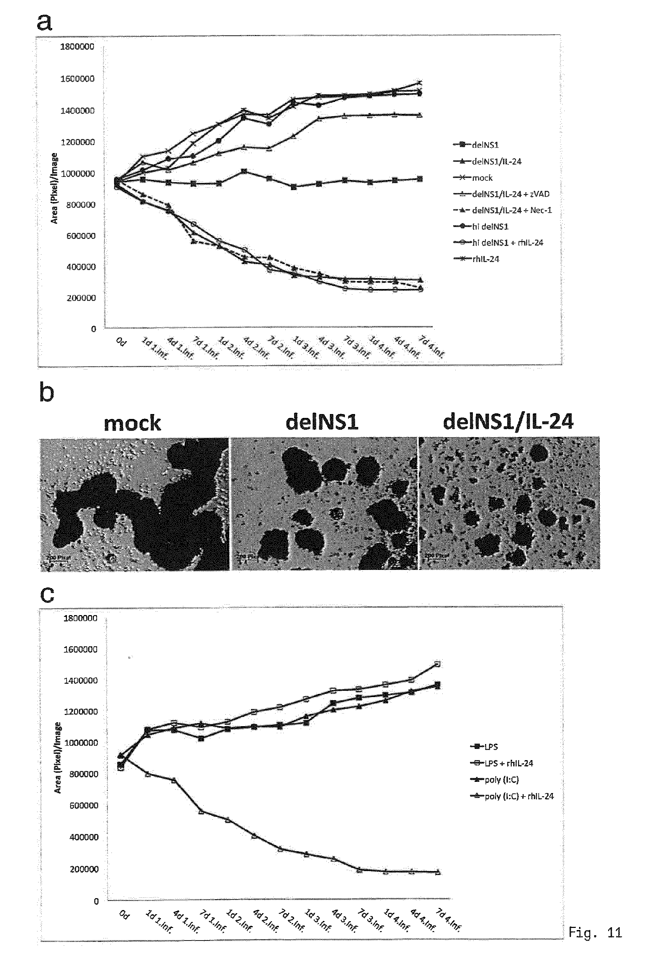

Figure 11 shows the effect of delNS1/IL-24 virus on spheroid formation of DU145 cells.(a) Spheroids were single infected with delNS1, delNS1/IL-24, heat inactivated (hi) delNS1 (moi=1) alone or in combination with recombinant human (rh) IL-24 (100ng/ml). In samples indicated cell zVAD or Nec-1 was added to the cultures. Re-infections of spheroids were performed weekly. Growth of DU145 spheroids was determined by ImageJ 1.440 software. (b) Representative images of spheroids treated with delNS1 and delNS1/IL-24 (moi=1) four days after 2nd infection are shown. (c) Spheroids were treated with LPS (100ng/ml) and poly (I:C)(2µg/ml) alone or in combination with rhIL-24.

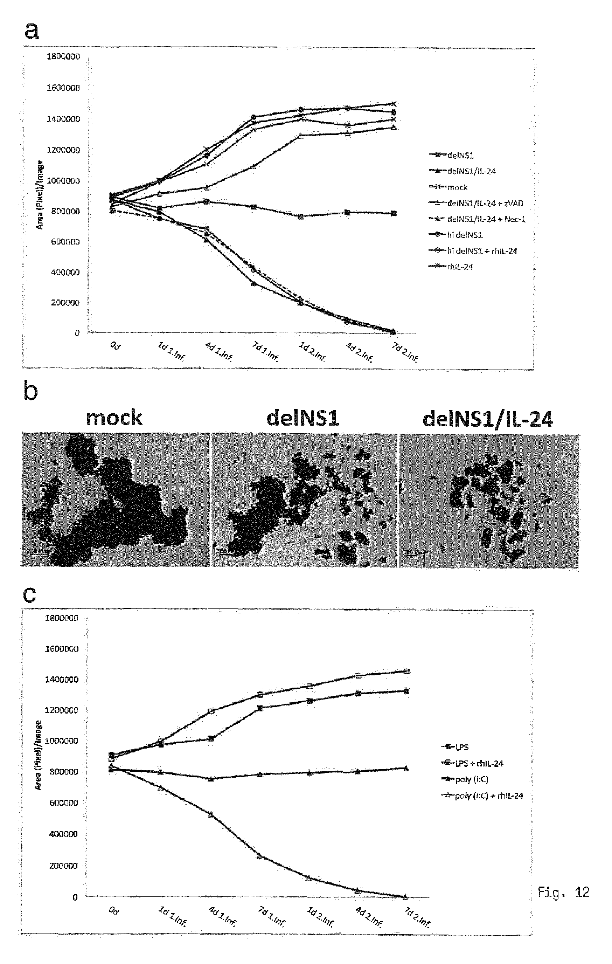

Figure 12 shows the effect of delNS1/IL-24 virus on spheroid formation of SK-Mel28 cells.(a) Spheroids were single infected with delNS1, delNS1/IL-24, heat inactivated (hi) delNS1 (moi=1) alone or in combination with recombinant human (rh) IL-24 (100ng/ml). In samples indicated cell zVAD or Nec-1 was added to the cultures. Re-infections of spheroids were performed weekly. Growth of spheroids was determined by ImageJ 1.440 software.(b) Representative images of DU145 spheroids treated with delNS1 and delNS1/IL-24 (moi=1) four days after 2nd infection are shown. (c) Spheroids were treated with LPS (100ng/ml) and poly (I:C)(2µg/ml) alone or in combination with rhIL-24.

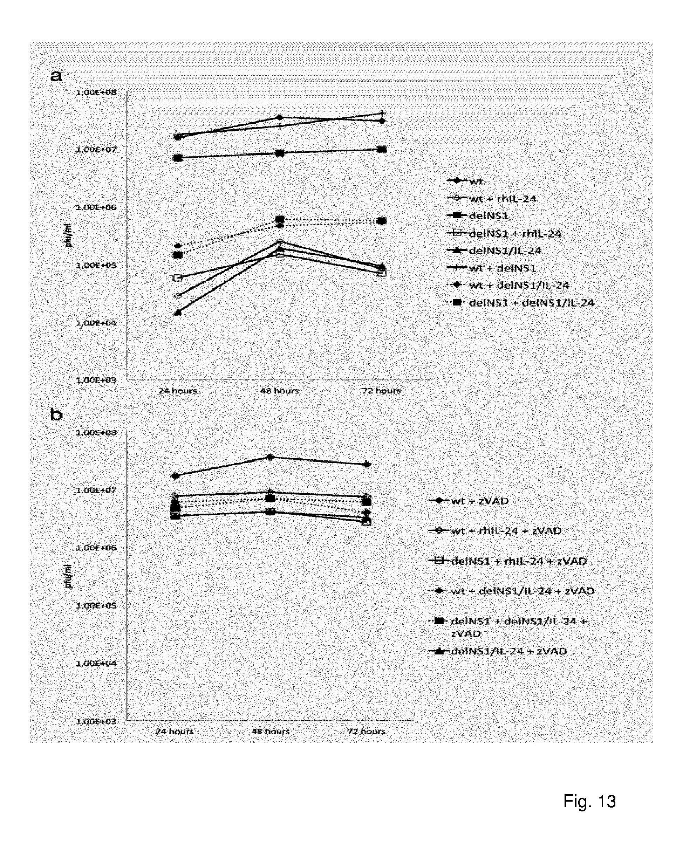

Figure 13 shows the effect of delNS1/Il-24 virus and recombinant IL-24 on growth of wild type virus in Vero cells with (a) and without (b) the addition of zVAD. Viruses used for infection and possible addition of IL-24 or the caspase inhibitor zVADare indicated on the right. Following multiplicity was used for infections. Wt: moi=0.01, delNS1 moi=0.1, delNS1/24 moi=0.1. wt, wild type virus; rhIL24, recombinant human interleukin 24, delNS1, virus lacking the non-structural protein 1, delNS1/IL-24, virus lacking the non-structural protein 1 but expressing IL-24.

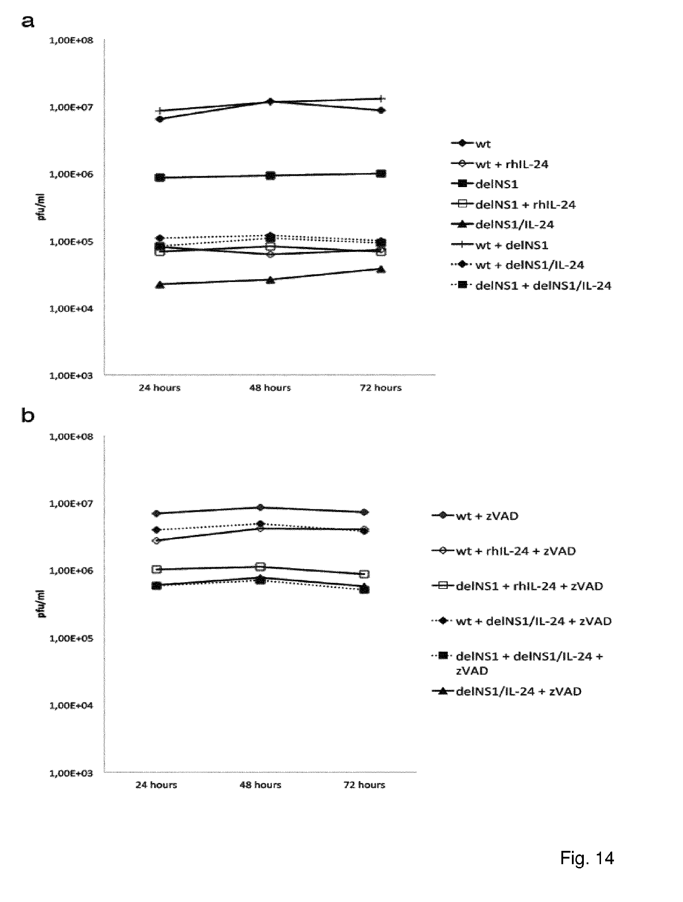

Figure 14 shows the effect of delNS1/Il-24 virus and recombinant IL-24 on growth of wild type virus in DU145 cells with (a) and without (b) the addition of zVAD. Viruses used for infection and possible addition of IL-24 or the caspase inhibitor zVAD is indicated on the right: wt, wild type virus; rhIL24, recombinant human interleukin 24, delNS1, virus lacking the non-structural protein 1, delNS1/IL-24, virus lacking the non-structural protein 1 but expressing IL-24.

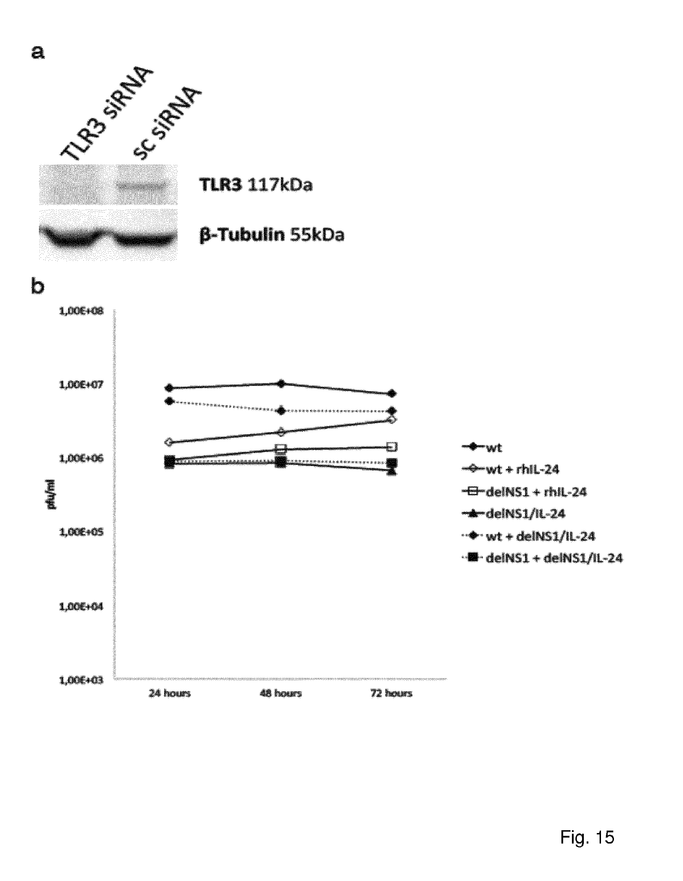

Figure 15 shows the effect of TLR-3 siRNA on a) TLR-3 protein expression and b) antiviral effects of IL-24. a) Western Blot analysis of TLR-3 expression. b) Effect of delNS1/Il-24 virus and recombinant IL-24 on growth of wild type virus in DU145 cells in the presence of TLR3 siRNA.

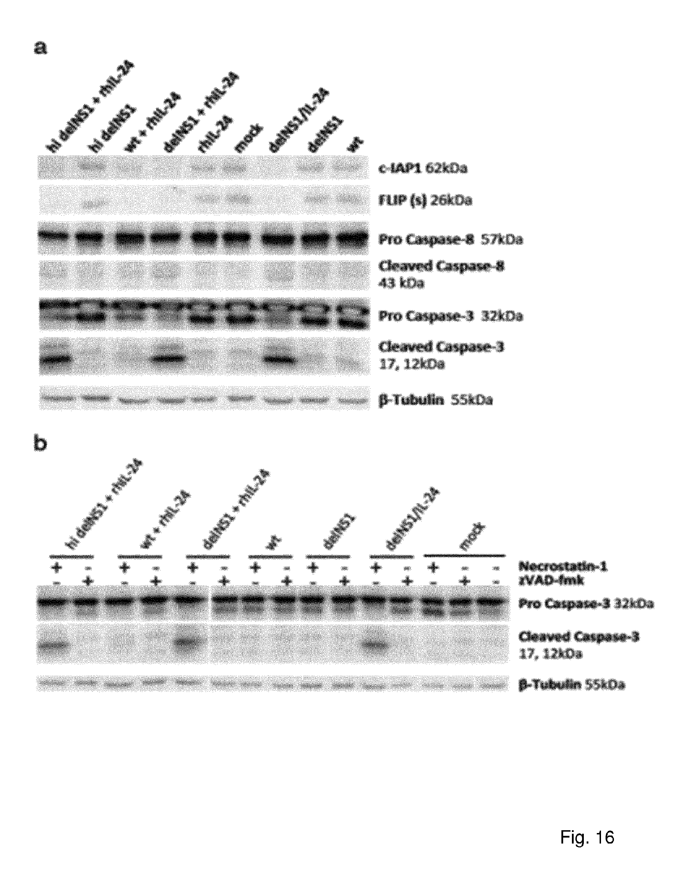

Figure 16 shows the effect of IL-24 in combination of viruses on apoptotic signal cascades without (a) and with (b) the addition of inhibitors of apoptosis (zVAD) and necroptosis (necrostatin-1) as determined by Western Blot analysis. The conditions of infection/stimulation by IL-24 and viruses are indicated on the top. Proteins analysed as indicated on the right.

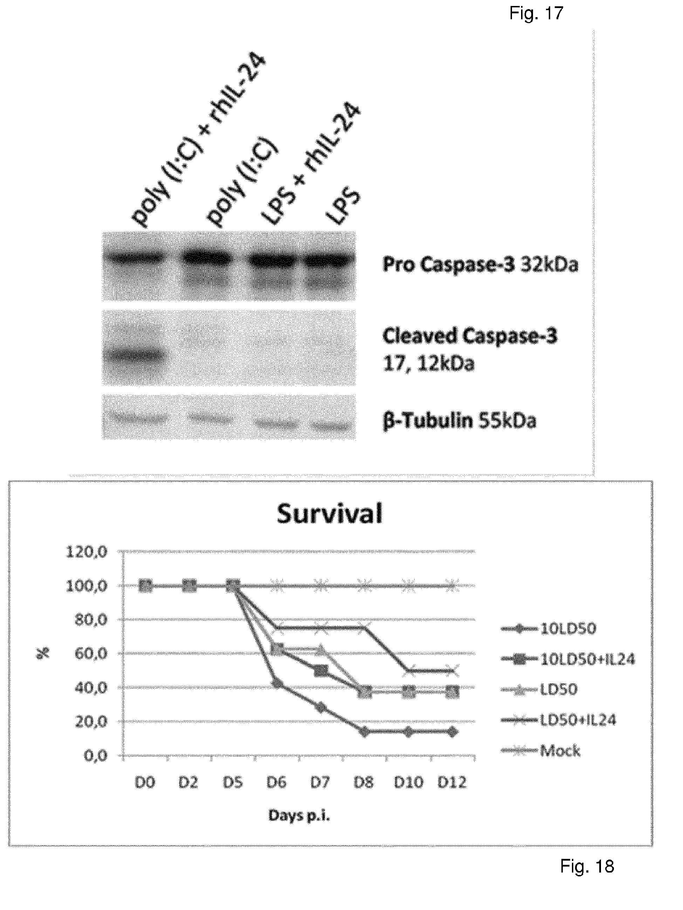

Figure 17 shows the effect of co-stimulation of recombinant human interleukin 24 with TLR 3 or TLR4 agonists on caspase activation in Vero cells as determined by Western blot analysis.
Figure 18 shows survival of outbred mice infected of the combination of wt virus and delNS1/24 virus. Viruses used for infection as indicated at the right. 10LD50, 10 lethal dosis 50 of wild type virus. LD50, 1 lethal dose 50 of wild type virus; IL24, delNS1/Il24, mock, SPGN buffer.

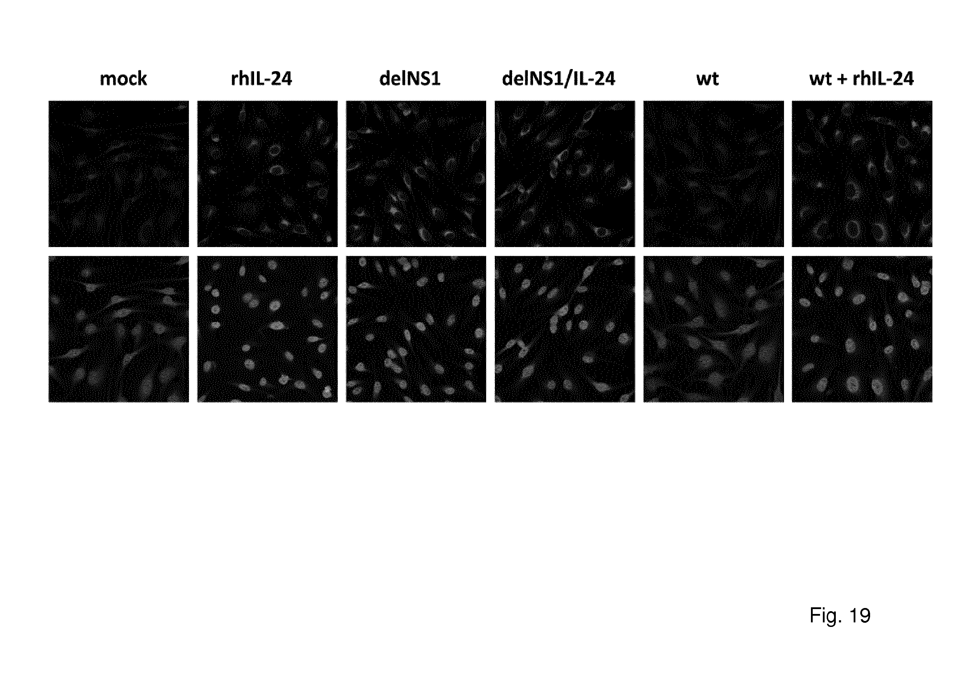

Figure 19 shows the effect of IL-24 and/or virus stimulation on PKR phosphorylation (red) and translocation of IRF3 into the nucleus (green) (16h; p.i.).

### Examples:

### 1. IL-24 and toll-like-receptor 3 activation synergize to induce apoptosis in tumor cells

Despite the fact that the physiological function of IL-24 remains unknown, its pro-apoptotic activity when expressed in adenovirus background is well documented, rendering IL-24 an attractive agent for anti-cancer virotherapy. Interestingly, when used as a single agent, IL-24 only induces apoptosis in certain tumor cell lines. Accordingly, within the course of the present invention, the oncolytic properties of an IL-24-expressing influenza A virus was analyzed. The analysis of the tumor-ablative potential of this chimeric RNA virus revealed that it induced a toll-like-receptor 3 (TLR3)-dependent apoptosis. Apoptosis was dependent on downregulation of the cellular FLICE-inhibitory protein (cFLIP), resulting in successive activation of caspases-8 and 3. Importantly, stimulation of TLR3 was found to be sufficient to promote IL-24 induced apoptotic cell death. This showed that the influenza A virus is an excellently suited vector to support the pro-apoptotic effect of IL-24 in cancer cells and proves that the combination of a TLR3-agonist and IL-24 is an effective tool for the treatment of cancer.

### Materials and methods (IL-24)

### Cell lines, Influenza Viruses and Reagents

The human melanoma cell line SK-Mel28 was purchased from ATCC (HTB-72) and was grown in DMEM/F12 medium (PAA, Pasching, Austria) supplemented with 10% heat-inactivated FCS. Human prostate cancer cell line DU145 was purchased from ATCC (ACC 261). Cells were maintained in a MEM medium (PAA, Pasching, Austria) supplemented with 10% heat inactivated FCS. VERO cells (EC ACC, 88020401), from kidney epithelial cells extracted from an African green monkey, adapted to grow on serum free medium were maintained in serum-free OPTIPRO Medium (Invitrogen, Carlsbad, US). The cells were maintained in a humidified 5% CO₂ atmosphere at 37°C.

IVR-116 wt, delNS1 (Wacheck et al., J. Infect. Dis. (2010), 354-62) and delNS1/IL-24(Wolschek et al., J. Virol. 85 (2011), 2469-2473)influenza viruses have been described previously. Heat-inactivation of delNS1 virus was performed at 56°C for 1 hour. For propagation of the viruses VERO cells were infected at a multiplicity of infection (m.o.i.) of 0,1 and incubated in OPTIPRO medium containing 5µg/ml trypsin (Sigma Aldrich, St. Louis, MO, US) at 37°C for 2 days. Virus concentrations were determined by plaque assay on VERO cells.

For stimulation experiments, cells were treated for 24 hours with recombinant human IL-24 (100ng/ml) (R&D Systems, Minneapolis, MN, US), 2-Val-Ala-DL-Asp-fluoromethylketone (z-VAD fmk) (Bachem, Torrance, CA, US), Necrostatin-1 (Nec-1) (Sigma-Aldrich, St. Louis, MO, US) and Resveratrol (Sigma-Aldrich, St. Louis, MO, US). The LPS used was ion-exchange chromatography-purified Escherichia coli LPS 055:B5 form Sigma Aldrich (St. Louis, MO, US), CL-097 and poly (I:C) from InvivoGen (San Diego, California, US). For stimulation experiment tumor necrosis factor alpha(TNFa)(R&D Systems, Minneapolis, MN, US), human interferon alpha (IFNα) (Health Protection Agency, London, UK) and human interferon gamma (IFNγ) (Imukin, BoehringerIngelheim, Vienna, Austria) were used in concentrations of 50 and 100 ng/ml supernatant, respectively.

### Cytokine Detection after Infection with Influenza Viruses

Cells were infected with viruses and cultured for 24h in medium containing 10% FCS. The supernatant was screened for human IL-24 by hIL-24 DuoSet ELISA (R&D Systems, Minneapolis, MN, US) according to the manufacturer's instructions.

### Cell Death Analysis using Annexin V/Propidium Iodide Double Staining Assay

Treated cells were collected after 24 hours, washed with phosphate buffered saline (PBS), and stained with 5µl fluorescein isothiocyanate (FITC)-conjugated Annexin V and PI for 15 minutes at room temperature in the dark (FITC Annexin V Apoptosis Detection Kit I, BD Pharmingen, Franklin Lakes, NJ, US). The stained cells were then analyzed by a flow cytometer (Gallios Cytometer 1.1, Beckman Coulter, Brea, California, US).

### Western Blot

Cells of interest were collected in RIPA sample buffer (Thermo Fisher Scientific, Rockford, IL, US) and stored at - 80°C. Samples were separated on a 12.5% denaturing acrylamide gel and transferred onto 0.45µm Protran nitrocellulose membrane (Whatman, Maidstone, Kent, UK). The membrane was blocked and incubated with rat monoclonal anti-c-IAP1 (1:1000) (Enzo Life Sciences, Farmingdale, NY, US), mouse monoclonal anti-FLIP (NF6) (1:500) (Enzo Life Sciences, Farmingdale, NY, US), mouse monoclonal anti-Caspase8 (1C12) (1:1000) (Cell Signaling, Danvers, MA, US), mouse monoclonal anti-Caspase3 (Enzo Life Sciences, Farmingdale, NY, US), mouse monoclonal anti-TLR3 (TLR3.7) (1:200) (Santa Cruz Biotechnology, Santa Cruz, California, US) or rabbit monoclonal anti-β-Tubulin (9F3) antibody (1:1000) (Cell Signaling, Danvers, MA, US) in TBS-Tween. Horseradish peroxidase (HRP) - conjugated anti-mouse IgG (1:1000) (Thermo Fisher Scientific, Rockford, IL, US), HRP-conjugated anti-rabbit IgG (1:1000) (Thermo Fisher, Rockford, IL, US) or HRP-conjugated anti-rat IgG (1:5000) (Enzo Life Sciences, Farmingdale, NY, US) were used as secondary antibodies. Positive bands were detected by chemiluminescence using a Super Signal West FemtoChemiluminescent Substrate (Thermo Fisher Scientific, Rockford, IL, US). Western Blot images were taken with a G:BOX (Syngene, Cambridge, UK) which is handled by the GeneSys software.

### Small interfering RNA (siRNA) Experiments

Cells were plated in six-well plates at 4×10⁴ cells. After overnight adherence 100µl serum- and antibiotic- free DMEM/F12 medium were mixed with 3µg/ml linear polyethyleneimin (Polysciences Europe GmbH, Eppelheim, Germany) and 100nM siRNA for 10 minutes on room temperature. Medium on the cells was changed to 900µl fresh DMEM/F12 medium (supplemented with FCS) and the previously mixed RNA solution was added to each well. Cells were incubated overnight and cultured for 72 hours in complete medium before treatment with viruses. TLR3 siRNA was purchased from Santa Cruz Biotechnology (Santa Cruz, California, US) and scrambled siRNA was from Qiagen (Maryland, US).

### Tumor spheroid cultures

Tumor spheres were prepared as previously described (Lopez et al., BMC Cancer 12 (2012), 48). In brief, cells were detached with EDTA (Life Technologies, Carlsbad, California, US), washed with PBS and placed in serum-free medium mixed with 20ng/ml epidermal growth factor (EGF PHG0311L, Life Technologies, Carlsbad, California, US), 20ng/ml basic fibroblast growth factor (bFGF PHG0021, Life Technologies, Carlsbad, California, US), and 0,4% bovine serum albumin (BSA, Sigma Aldrich, St. Louis, MO, US) at a density of 1 × 10³ cells/ml and cultured in ultra-low attachment plates (CLS3473 Costar, Sigma Aldrich St. Louis, MO, US). The sphere culture medium was changed every 48h until the majority of spheres reached 300-400µm in diameter.

### Effect of IL-24 and/or virus stimulation on PKR phosphorylation

24 hours old monolayer of SK-Mel28 cells was infected with wild-type (wt), delNS1, delNS1/IL-24 virus at an MOI=3 in the presence or absence of recombinant human (rh) IL-24 (100ng/ml). At 16 hours post-infection (p.i.) cells were subjected to the immunofluorescence assay with IRF3 and pPKR-specific antibodies.

### Microscopy

Spheroid cultures were photographed on a Zeiss Axiovert 40 CFL microscope (Zeiss, Jena, Germany). For each condition, four different fields were captured and representative images were selected. All images were digitally processed for presentation with Adobe Photoshop CS4.

### Statistical Analysis

All data are represented by the mean and SEM of at least three independent experiments. Statistical analysis was performed using the statistical software SPSS Statistics 17.0.0 (SPSS Software, Armonk, NY) and with GraphPadPrism Version 5 (GraphPad Software, La Jolla, CA).

### Co-Infection experiments

Cells were infected with viruses and stimulating adjuvants and cultured for 72 hours in OPTIPRO medium containing 5µg/ml trypsin-EDTA (Sigma Aldrich, St. Louis, MO, US). Every 24 hours supernatant was taken and virus concentrations were determined by plaque assay on VERO cells.

### Murine coinfection experiments:

For murine co-infection experiments female outbred mice (age 10-12 weeks) were infected under narcosiswith A/PR8/34 virus alone or in combination with the delNS1/24 virus according to following schedule:

| **Group** | **No. of animals** | **Immunization Virus** | **Virus titre** | **Dilution** | **Route, volume** |
|---|---|---|---|---|---|
| 1 | 7 | A/PR/8/34 10LD50 | 1,78*10⁹ EID₅₀/ml | 1:200 | i.n., 0,05 ml |
| 2 | 8 | A/PR/8/34 10LD50 | 1,78*10⁹ EID₅₀/ml | 1:100 | i.n., 0,05 ml |
| | | +delNS1/IL24 | 2,82*10⁶ | nd | |
| | | | TCID₅₀/ml | | |
| 3 | 8 | A/PR/8/34 LD50 | 1,78*10⁹ EID₅₀/ml | 1:2000 | i.n., 0,05 ml |
| 4 | 8 | A/PR/8/34LD50 | 1,78*10⁹ EID₅₀/ml | 1:1000 | i.n., 0,05 ml |
| | | +delNS1/L24 | 2,82*10⁶ | nd | |
| | | | TCID₅₀/ml | | |
| 5 | 6 | Mock (OptiOro) | - | - | i.n., 0,05 ml |

### Survival was recorded daily and determined up to day 12

### Results

### Characterization of viral growth and transgene expression of delNS1/IL-24 virus

Using a conditionally replicating influenza A viral vector expressing the secreted form of IL-24 (delNS1/IL-24 virus), which was previously constructed (Wolschek et al., J. Virol. 85 (2011), 2469-2473), the replication properties of influenza wild-type (wt) virus, empty vector delNS1 virus and the newly-derived delNS1/IL-24 virus was compared. Similar to the delNS1 virus, delNS1/IL-24 virus retained the ability to grow on both, a prostate- and a melanoma-derived tumor cell line (DU145 and SK-Me128). As expected, the growth of the NS1 deletion viruses was reduced when compared to wt virus. DelNS1 virus also grew in the producer cell line VERO. Virally expressed IL-24 was produced to concentrations of up to 6 ng/ml in the cell lines infected with the chimeric virus (Tablel).

**Table 1: Comparison of viral growth and transgene expression in different cell lines**

| | | **pfu/ml** | **rhIL-24 (pg/ml)** |
|---|---|---|---|
| **DU145** | | | |
| | delNS1 | 2.9×10⁵ | <10° |
| | | 1.3×10⁴ | 6.3×10³ ± 0.3 |
| delNS1/IL-24 | | 1.0×10⁷ | <10° |
| | wt | | |

| **SKMe128** | | | |
|---|---|---|---|
| | delNS1 | 5.1×10⁴ | <10° |
| | | 3.8×10⁴ | 2.1×10³ ± 0.2 |
| delNS1/IL-24 | | 5.6×10⁵ | <10° |
| | wt | | |

### DelNS1/IL-24 virus induced apoptosis in tumor cells

It is known, that NS1 deletion viruses induce higher amounts of apoptosis than wt virus infection (Zhirnov et al., J.Virol.76 (2002), 1617-25). It was therefore analyzed, whether expression of IL-24 by a delNS1 would further enhance apoptosis in DU145 cells (Fig.1) or SK-Mel28 cells (Fig. 2) using AnnexinV/PI-staining for detection of cell death. Infection of DU145 cells with delNS1/IL-24 virus significantly increased the amount of early and late apoptotic/necrotic cells to 36.4% (p=0.03) as compared to infection with empty delNS1 virus. According to the literature, the delNS1 virus vector induces higher levels of apoptosis than wt virus (21.0% versus 12.6%, respectively). Thus, IL-24 could further enhance delNS1-associated apoptosis. Importantly, when wt or delNS1 virus was combined with 100ng of recombinant human (rh)IL-24, apoptotic/late necrotic cells were induced up to 35.8% (p=0.001) and 42.9% (p=0.01), respectively as compared to viral stimulation alone. This showed that intracellular synthesis or autocrine action of the cytokine was not required for the pro-apoptotic effect. Interestingly, there was no increase in apoptosis after adding rhIL-24 to either cell line alone. The presence of the virus therefore provided an essential second stimulus for the induction of IL-24-mediated apoptosis.

Next it was therefore aimed to elucidate this second stimulus, which promoted apoptosis of IL-24. It is known, that structural parts of the influenza A viruses particles such as viral RNA or the interaction of the viral entry protein with the cellular receptor can activate TLR-3 and TLR-7 receptors). As activation of TLRs was shown to promote apoptosis under appropriate conditions, it was tested, whether a structural part of the virus possibly directly enables cell death induced by IL-24. Indeed, it was found that infection of DU145 cells with a heat-inactivated (hi delNS1) virus, which can bind to the viral receptor but cannot replicate in the cell, also increased the amount of early and late apoptotic/necrotic cells up to 41% (p=0.002) after adding rhIL-24. Thus, presence of the virus particle itself appeared to be essential to enable IL-24-induced apoptosis in these tumor cells. Largely similar results were obtained with the melanoma cell line SK-Mel28.

### IL-24/influenza A virus-induced cell death is apoptotic and not necroptotic

Most recently, it was shown, that activation of TLR3 induces a necroptotic cell death when combined with smacmimetics. To differentiate between apoptotic and necroptotic cell death, the effect of caspase inhibitor zVAD-fmk (zVAD) and the effect of necrostatin-1 (Nec-1) on influenza virus/IL-24 induced cell death was tested. Nec-1 is an inhibitor of the kinase RIP1, which initiates necroptosis. No decrease in the amount of early, late apoptotic/necrotic cells could be observed after single infection of DU145 cells with wt and delNS1 virus by the use of zVAD and Nec-1 alone (Fig.1 and Fig.2). In contrast, infection of cells with delNS1/IL-24 or the combination of wt, delNS1 and hi delNS1 plus rhIL-24 the addition of zVADrescued all cells from cell death (p=0.03 for delNS1/IL-24 versus delNS1/IL- 24 +zVAD, p=0.01 for wt + rhIL-24 versus wt + rhIL-24 +zVAD, p=0.008 for delNS1 + rhIL-24 versus delNS1 + rhIL-24 +zVAD, p=0.03 for hi delNS1 + rhIL-24 versus hi delNS1 + rhIL-24 +zVAD). No decrease in the amount of early, late apoptotic/necrotic cells could be found after treatment with Nec-1.

### Apoptotic signaling pathways are induced by delNS1/IL-24 virus

TLR-mediated induction apoptosis was shown to be mediated via activation of caspase-8 and caspase-3. Moreover, the cellular inhibitor of apoptosis 1 (cIAP1) and both the long and short isoforms of the cellular FLICE-like inhibitory protein (cFLIP), cFLIP_{L} and cFLIP_{S}, can play decisive roles for induction of apoptosis versus necroptosis. To further elucidate the mechanisms of cell death induction during influenza virus/IL-24 stimulation, the expression/activation of those molecules was monitored following treatment of cells with delNS1/IL-24 or the combination of wt, delNS1 or hi delNS1 and rhIL-24 (Fig. 3a and Fig.4a). Infection of DU145 cells with delNS1/IL-24 or combined treatment of cells with delNS1 and rhIL-24 or hi delNS1 and rhIL-24 led to a decrease of cIAP1 protein expression level. Similarly, a decrease in the expression of anti-apoptotic proteins such as cFLIP could also be observed after infection with delNS1/IL-24 and combined infection of delNS1, wt virus, hi delNS1 plus rhIL-24 but not after infection with non-chimeric virus or by adding rhIL24 alone. It should be noted that samples stimulated with wt virus and rhIL24 protein did show a reduction of cFLIP_{L} and cFLIP_{S}, despite the fact that cIAP1 expression was not altered. Concordant with the downregulation of cFLIP_{L} and cFLIP_{S} the cleaved forms of caspase-8 and caspase-3 increased following single infection of delNS1/IL-24 virus and combined treatment of wt, delNS1, hi delNS1 with 100ng rhIL-24 protein as compared to single infections with these viruses or mock infection.

Again, corresponding effects of the combined virus/IL-24 treatment were observed in SK-Mel28 cells with respect to regulation of both cFLIP isoforms and caspase activation. It should be noted that in samples stimulated with wt virus and rhIL-24, caspase protein expression levels were not as high as in cells infected only with delNS1/IL-24 virus or in which infection with delNS1, or treatment with hi delNS1 was combined with addition of rhIL-24. Expression of cIAP1 was more strongly negatively affected by virus/IL-24 stimulation in SK-Mel28 cells than in DU145 cells.

To further rule out the induction of necroptosis in the present system the effect of zVAD or Nec-1 on caspase-3 activation was analyzed. zVAD blocked cleavage of caspase-3 after infection with delNS1/IL-24 or the combined stimulation of cells with wt, delNS1 or hidelNS1 and rhIL-24. In contrast, no decrease in the levels of cleaved caspase-3 protein was found in any of the cases of treatment with Nec-1. Similar effects were observed on the melanoma cell line SK-Mel28 (Fig. 4b).

### TLR3 signaling enhances IL-24-mediated apoptosis

Next it was aimed to investigate which toll like receptor(s) mediate(s) the contribution of viral particles to IL-24-induced apoptosis in DU145 (Fig. 5) and in SK-Mel28 cells (Fig. 6). As mentioned above, influenza A viruses are known to stimulate toll-like receptor 3 (TLR3) and TLR7 which usually sense foreign RNA. Therefore both, a TLR3-agonist (poly (I:C)) and a TLR7 agonist (CL-097) were tested in combination with rhIL-24 in the present setting. In order to rule out non-specific TLR activation stimulation by LPS, known to activate TLR2 and TLR4, was included as a control in these experiments. TLR4 was also used, as TLR4 mediated apoptosis has previously been described. Single treatment of DU145 cells with any of the three agonists did not increase the amount of early and late apoptotic cells. In contrast, when combined with rhIL-24, stimulation of TLR3, but not of TLR2,4, or 7, resulted in highly effective induction of apoptosis (Fig.5a) and caspase-3 cleavage (Fig. 5b) in the tumor cells treated with this combination. Hence, stimulation of TLR3 is sufficient to enhance apoptosis mediated by IL-24.

To further substantiate the role of TLR3 stimulation in IL-24-mediated apoptosis two different approaches to inhibit TLR3 signaling were employed. First it was interfered with TLR3 signaling using a siRNA mediated knockdown that reduced TLR3 protein levels by more than 80% (Fig 5a). Knockdown of TLR3 completely prevented caspase-3 cleavage induced by single infection with delNS1/IL-24 as compared to cells treated with a control knockdown. The same, in cells stimulated with the combination of delNSl, hi delNS1, or poly (I:C) with rhIL-24 cleavage of caspase-3 was also prevented by TLR3 knockdown as compared to a control knockdown (Fig. 7b). To further verify a TLR-mediated effect in the present system a pharmacological inhibition of TLR signaling using resveratrol (3,4',5-trihydroxy-trans-stilbene), a phenolic phytoalexin found in grape skin and other plants, was used. Resveratrol suppresses MyD88-independent, but not MyD88-dependent signaling pathways of TLR3 and TLR. 50µM of resveratrol decreased the amount of early and late apoptotic cells from 40.8%±0.8 to 22%±1.4 in delNS1/IL-24 virus infected DU145 cells (p=0.05) (Fig. 8). A similar decrease was found when resveratrol was added to samples stimulated with rhIL-24 in combination of delNS1 (40.5%±0.6 versus 20.5%±0.7, p=0.03), hi delNS1 (41.4%±0.1 versus 19.5%±0.7, p=0.02) or poly (I:C) (66.6%±1.0 versus 46.0%±2.4, p=0.03). In contrast no decrease in apoptosis was observed after single infection with delNS1, hi delNS1 and poly (I:C) alone by the presence of resveratrol. Similar observation were done on SK-Mel28 cells (Fig. 9 and Fig. 10)

### The combination of rhIL-24 with virus or poly (I:C) abolishes established tumor spheroid cultures

Tumor spheroids can serve as a readily accessible three dimensional, and as such more physiological, in-vitro model of tumor formation and growth. Therefore, spheroid cultures of DU145 tumor cells and SK-Mel28were established. Infection of spheroid cultures with delNS1/IL-24 dramatically reduced their growth (Fig. 11 and Fig. 12). In contrast, the empty delNS1 only inhibited further outgrowth of spheroids. RhIL-24 alone had no effect at all and was comparable to mock treatment. Corresponding to the 2-dimensional cell cultures, the combination of rhIL-24 with hi delNS1 achieved the same level of growth reduction than infection of spheroids with the chimeric delNS1/IL-24 virus. The apparent reduction in growth by delNS1/24 virus was again almost completely inhibited by the caspase inhibitor zVAD but not by Nec-1. As the essential role of TLR3 for IL-24 induced apoptosis was identified in the 2-dimensional models also the effect of poly (I:C) and rhIL-24 in a spheroid cultures was tested. The combination of LPS and rhIL24 was used as a negative control to rule out unspecific immune-mediated effects. Inhibition of growth was only observed for spheroids treated with poly (I:C) in combination with rhIL-24 but in any of the other groups such as LPS plus rhIL-24 or any agent alone (Fig. 6c).

### Effect of IL-24 and/or virus stimulation on PKR phosphorylation and translocation of IRF3 into the nucleus.

A strategy to fight viral infections is the reinforcement of the antiviral cellular pathways, which are downregulated by viral pathogenicity factors. Those pathways include activation of PKR and the translocation of IRF3 into the nucleus.

In Fig. 19 it is shown that IL-24 has an activating effect on phosphorylation of PKR molecules and positively regulating translocation of IRF3 into the nucleus of non-infected and infected with wt influenza A virus cells.

### Discussion

Analyzing the tumor ablative potential of a chimeric influenza virus expressing IL-24 a potent synergistic activity of IL-24 when combined with TLR3 stimulation was shown by the present examples. This shows that i) effective IL-24-induced apoptosis might frequently be dependent on an appropriate second signal, and that ii) armed oncolytic influenza viruses can provide such a second signal.

Stimulation of TLR3 or TLR4 had previously been shown to be capable of promoting caspase-8/3-dependent apoptosis, however, only in the presence of cycloheximid. Here it was found that in the case of TLR3 activation, a very similar outcome can also be achieved by a protein encoded in the human genome, namely IL-24, as this cytokine sensitizes tumor cells to a TLR3-activation-induced apoptosis that depends on activation of caspases-8 and 3. Despite the fact that the same was not observed when stimulating TLR4 in the presence of IL-24, this observation suggests that similar mechanisms might be at work for IL-24 and CHX-mediated sensitization to TLR-mediated apoptosis. CHX-mediated sensitization to TLR4-activation-induced apoptosis was shown to be due to downregulation of cFLIP_{L}. Similarly, cFLIP_{L} is also inhibited in TLR3-induced apoptosis in dendritic cells. In line with the notion that a similar mechanism of sensitisation might be operative in both scenarios, it was found here also that cells that undergo IL-24/TLR3-mediated apoptosis, levels of cFLIP_{L} are reduced. Thus, cFLIP_{L} down regulation may also be a critical step for apoptosis induction by IL-24/TLR3 activation. Previously, IL-24 has been described to induce phosphorylation of PKR which is associated with eIF2α phosphorylation which in turn blocks translation. Moreover, IL-24 promoted tumor cell death can also be dependent on PERK, another activator of eIF2α phosphorylation. Both, the long and the short isoforms of cFLIP have previously been shown to be highly unstable and that for their continuous presence they need to be continuously produced. It is therefore likely that the apoptosis-enabling effect of IL-24 on TLR3 signaling may be due to the translation-inhibitory function of IL-24 in general and, more specifically, the one exerted on the translation of cFLIP.

Previous studies found that in selected cell lines the apoptosis-promoting effect of TLR3 activation was at least partially dependent on the type I IFN response status. As DU145 cells are IFN-resistant, but sensitive to IL-24/TLR3-activation-mediated apoptosis, it is clear that IFN is not required for this type of cell death. It is, however, possible that type I IFN could serve as a cofactor in IL-24/TLR3-activation-mediated apoptosis due to following observation: rhIL24 did not enhance apoptosis when combined with wt virus in SK-Mel28 cells, but rhIL24 enhanced apoptosis when combined with the delNS1 virus, the latter which is known to induce much higher levels of type I IFN, since its RNA is not neutralized by the presence of the viral NS1 protein.

Most recently it was disclosed that, under certain circumstances, TLR3 stimulation resulted in activation of caspase-8 in the subsequent induction of necroptosis. Despite the fact that IL-24/TLR-3 co-stimulation also prevented transcription of cFLIP_{L} - which inhibits ripoptosome formation -, IL-24-enabled TLR3-activation-induced cell death could not be blocked with Nec-1 whilst the broad spectrum caspase inhibitor zVAD was very efficiently capable of doing so. Taken into consideration that the application of zVAD usually further sensitizes cells to necroptosis or is even required to enable this form of cell death, the findings that zVAD completely inhibits and Nec-1 does not alter the course of cell death induction by IL-24-TLR3 activation, indicates that the form of cell death induced by this combination is in its majority, if not entirety, due to apoptosis and not necroptosis.

For therapeutic use in cancer treatment neither systemic application of IL-24 nor systemic application of poly (I:C) might be appropriate due to possible toxicity. Thus, one option to ensure effective therapeutic levels in the tumor microenvironment is the use of an armed oncolytic virus, such as the previously constructed conditionally replicating influenza A virus which expresses the secreted version of IL-24 (Wolschek et al., J. Virol. 85 (2011), 2469-2473). As influenza A virus is known to stimulate TLR3 it is a highly suitable viral vector for delivery of IL-24 in cancer therapy. In the present study it was determined that TLR3 signaling is crucial for therapeutic effects exerted by armed influenza viruses, as both pharmacological inhibition of MyD88-independent TLR3 signaling by resveratrol and RNA-interference-mediated suppression of TLR3 expression inhibited IL-24/influenza A-virus-induced apoptosis. It was also shown that the combination of influenza A virus with rhIL-24 was as effective as with virally expressed IL-24. This shows that IL-24 acts in its secreted form and can therefore also exert a bystander effect, i.e. also act on non-productively infected cells. Along the same lines, it was determined that the viral component of the synergistic effect of IL-24 and influenza A virus to be stimulation of TLR3, and that this is not only induced by infectious particles but also by non-infectious particles acting, which can again act on non-infected neighboring cells. Thus, using a chimeric IL-24-expressing influenza A virus does not only promote killing of infected tumor cells but also delivers a maximal bystander effect as both active components required for the synergistic induction of apoptosis are readily available to cells surrounding the infected cells. Accordingly, the effective tumor ablation was demonstrated in spheroid cultures of the prostate carcinoma cell line DU145 as well as of the human melanoma cell line SK-Mel28 by the combination of influenza A virus and IL-24.

Of note, the viral vector used in the present examples is known to induce high levels of PKR activation. Importantly, the wt virus - which does not increase the level of PKR activation - also induced apoptosis in combination with rhIL-24 in DU145 cells. This demonstrates that neither the vector backbone-induced PKR activation is substantial in these tumor cells. This could be explained by the fact that IL-24 by itself is known to induce PKR phosphorylation, rendering virally induced PKR less relevant.

Whereas its physiological role remains to be determined, the pro-apoptotic activity of IL-24 on tumor cells has recently gained increasing attention. It should, however, be noted that in these studies the pro-apoptotic function of IL-24 was usually investigated by the means of cytokine-expressing adenoviruses. Intriguingly, the use of IL-24 as a single agent gave conflicting results regarding its pro-apoptotic effect. The present data now show that by inhibiting the expression of cFLIP, and perhaps other short-lived anti-apoptotic proteins which remain to be discovered, IL-24 acts as a sensitizer for apoptosis induction by a second stimulus which, in the case of the present study, is provided by activation of TLR3. Hence, the observed differences in reactivity of tumor cell lines following stimulation with IL-24 might very well be due to the presence or absence of such a second signal and activation of the respective downstream signaling pathway. On the flip side of the same coin, however, the fact that adenovirus-expressed IL-24 always induces apoptosis suggests that adenovirus also inherently induces an appropriate second signal. As adenovirus is known to primarily stimulate TLR2 and TLR9, induction of IL-24-enabled apoptosis is likely not to be restricted to stimulation of TLR3.

In conclusion, it was shown with the examples of the present invention that IL-24 enables TLR3-induced apoptosis. On the one hand, the present findings might hint at the physiological function of IL-24, as this cytokine could contribute to the elimination of infected or activated cells by apoptosis during the course of an inflammation. On the other hand, and with regards to the development of novel therapeutic options in cancer, the synergistic capacity of cell death induction by IL-24 and TLR3-agonists described here, identifies oncolytic RNA viruses as especially suitable for the expression of this cytokine. In addition, it shows the suitability of IL-24/TLR3 ligand combinations for the treatment of cancer.

### 2. Antiviral effect of IL-24, which is mediated by co-stimulation of TLR3 by the viral infection.

### Results

The IL-24 ORF was expressed with an influenza A virus, in which the NS1 protein was deleted (delNS/IL-24 virus) (Wolschek et al, J. Virol. 2011). First the growth of this virus and its IL-24 expression were determined in the IFN resistant influenza virus producer cell line Vero and in the prostate carcinoma cell line DU 145 (Table 2).

**Table 2**

| | | pfu/ml | rhIL-24 (pg/ml) |
|---|---|---|---|
| DU145 | | | |
| | delNS1 | 2.9 x 10⁵ | < 10⁰ |
| | delNS1/IL-24 | 1.3 x 10⁴ | 6.3 x 10³± 0.3 |
| | wt | 1.0 x 10⁷ | < 10⁰ |
| | | | |

| VERO | | | |
|---|---|---|---|
| | delNS1 | 1.2 x 10⁷ | < 10⁰ |
| | delNS1/IL-24 | 2.5 x 10⁵ | 8.4 x 10³± 0.4 |
| | wt | 3.6 x 10⁷ | < 10⁰ |

It was then observed, that a virus expressing IL-24 reduced the titer of corresponding wild type IVR116 virus (wt) (Fig.13a) in Vero cells, which are defective for IFN. The empty viral vector (delNS1 virus) did not reduce the titer of wild type virus when used for co-infection. The addition of IL-24 in a concentration of 100ng/ml was sufficient to promote the antiviral effect.

This shows that IL-24 by itself and IL-24 expressed by a chimeric virus as an antiviral effect. The expression of IL-24 by a chimeric virus will have the advantage of local expression of high concentrations of the cytokine in the area of disease. As Vero cells are defective in IFN signalling this suggests that type I IFN does not play a role in this setting.

The addition of the caspase inhibitor zVAD abolished the effect of IL-24, indicating that IL-24 mediated antiviral activities require the induction of caspases. The addition of zVAD increased the titer of the IL-24 expressing viruses. This indicates that zVADcan be used as an additive for the growth of IL-24 expressing viruses to increase their yield.

Next the effect if IL-24 on the growth of wild type virus on prostate carcinoma cell line DU145was determined. This cell line is known to be highly responsive to IL-24. Moreover, this cell lines is less IFN defective than Vero cells used above. Corresponding to the data obtained in Vero cells (Fig.13), recombinant human IL-24 or IL24 expressed by the delNS/IL24 virus again reduced the growth of wild type virus by approximately two logs (Fig. 14).

Next it was investigated, whether virus induced activation TLR-3 is involved in the antiviral effect of IL-24, as it was previously shown that IL-24 exerts its pro-apoptotic activity only if TLR-3 is stimulated. Therefore, TL-3 signalling was blocked by an TLR-3 antisense. Fig. 15a demonstrates the reduced expression of TLR3 following TLR3 si-RNA treatment. Moreover, TLR siRNA treatment completely abolished the antiviral effect of delNS1/IL24 virus and reduced the antiviral effect of rhIL-24 to one log (Fig 15b).

Previously it was demonstrated, that the induction of apoptosis by IL-24/TLR3 co-stimulation is i) dependent on activation of caspase 8 and caspase 3 and ii) correlates with a downregulation of cFLIP(s) and cIAP1 expression in tumor cell lines. Here the activation of the above described pathway is also demonstrated in Vero cells (Fig. 16a); further indicating that type I IFN has no effect in this system. Moreover it was demonstrated that the IL-24/TLR3 stimulated activation of caspase 3 can be blocked by zVAD (Fig. 16b), but not by necorstatin-1, which further demonstrates that this combination induces apoptosis but not necroptosis.

It was next determined, whether IL-24 also induces an apoptotic event in the presence of a pure TLR-3 stimulation without the presence of a virus. Vero cell were treated by the TLR3-agonistpolyIC in combination with IL-24. Then the activation of caspase 3 as a marker for apoptosis was determined (Fig. 17). Co-stimulation of IL-24 and the TLR4 stimulus was used as an unspecific control. The data show that only TLR3 stimulation, but not TLR4 stimulation induced an activation of caspases in the IFN defective Vero cells.

Next the effect of delNS1/Il-24 virus on growth of wild type viruses in vivo was tested. For this purpose groups of mice were coinfected with under the regimen shown in Table 3.

**Table 3: Groups, animal numbers, dose levels used for co-infection experiments in mice in vivo**

| Group | No. of animals | Immunization Virus | | Virus titre | Dilution | Route, volume |
|---|---|---|---|---|---|---|
| 1 | 7 | A/PR/8/34 10LD 50 | | 1,78*10⁹ EID₅₀/ml | 1:200 | i.n., 0,05 ml |
| 2 | 8 | A/PR/8/34 10LD50 + delNS1/IL24 | | 1,78*10⁹ EID₅₀/ml 2,82*10⁶ TCID₅₀/ml | 1:100 nd | i.n., 0,05 ml |
| 3 | 8 | A/PR/8/34 LD50 | | 1,78*10⁹ EID₅₀/ml | 1:2000 | i.n., 0,05 ml |
| 4 | 8 | A/PR/8/34LD50 +delNS1/IL24 | | 1,78*10⁹ EID₅₀/ml 2,82*10⁶ TCID₅₀/ml | 1:1000 nd | i.n., 0,05 ml |
| 5 | 6 | Mock (OptiOro) | | - | - | i.n., 0,05 ml |

Survival analysis shows that the addition of delNS1/Il24 virus to wild type virus decreased the lethal potential of the latter (Fig. 18). It should be noted that infection of mice with delNS1/24 was to comparable attenuation than the delNS1 vaccine virus.

## Claims

1. Pharmaceutical combination of
- a TLR3-agonist and
- Interleukin 24 (IL-24).

2. Pharmaceutical combination of
- a TLR3-agonist and
- Interleukin 24 (IL-24) for use in the treatment of cancer or for the treatment of viral infections.

3. Pharmaceutical combination according to claims 1 or 2 wherein the TLR3-agonist is selected from double-stranded RNA (dsRNA), especially poly(A:U)-dsRNA and poly(I:C)-dsRNA.

4. Pharmaceutical combination according to any one of claims 1 to 3 wherein the combination is present in a single pharmaceutical preparation.

5. Pharmaceutical combination according to any one of claims 1 to 3 wherein the TLR3-agonist and the IL-24 are provided in separate pharmaceutical preparations.

6. Pharmaceutical combination according to any one of claims 1 to 5 further comprising a pharmaceutically acceptable excipient.

7. Pharmaceutical combination according to any one of claims 1 to 6 wherein the combination is provided as an implantable drug release device.

8. Pharmaceutical preparation comprising a viral vector being a TLR3-agonist and being capable of expressing IL-24.

9. Pharmaceutical preparation comprising a viral vector being a TLR3-agonist and being capable of expressing IL-24 for use in the treatment of cancer or for the treatment of viral infections.

10. Pharmaceutical preparation according to claims 8 or 9 wherein the viral vector is an influenza virus vector, preferably an influenza A virus vector.

11. Pharmaceutical combination according to any one of claims 1 to 7 or pharmaceutical preparation according to any one of claims 8 to 10, wherein the IL-24 is human recombinant IL-24.

12. Pharmaceutical combination according to any one of claims 1 to 7 and 11 or pharmaceutical preparation according to any one of claims 8 to 11 for use in a method for treating a cancer patient or for the treatment of a patient suffering from a viral infection comprising administering an effective amount of the pharmaceutical combination or the pharmaceutical preparation.

13. Pharmaceutical combination according to any one of claims 1 to 7 and 11 or pharmaceutical preparation according to any one of claims 8 to 11 for use in a method for treating a cancer patient or for the treatment of a patient suffering from a viral infection wherein the administering of the combination or the preparation is performed by administering a single dosage to the cancer patient or the patient suffering from a viral infection.

14. Pharmaceutical combination according to any one of claims 1 to 7 and 11 or pharmaceutical preparation according to any one of claims 8 to 11 for use in a method for treating a cancer patient, wherein a single dosage of the TLR3-agonist and IL-24 is administered to the cancer patient or for the treatment of a patient suffering from a viral infection and the single dosage, independently of each other, is 0.1 to 100 mg/kg body weight, preferably 0.5 to 50 mg/kg body weight, especially 1 to 10 mg/kg body weight, of the TLR3-agonist, and 0.01 to 10000 ng/ml, preferably 1 to 1000 ng/ml, especially 30 to 300 ng/ml, of IL-24.

15. Pharmaceutical combination according to any one of claims 1 to 7 and 11 or pharmaceutical preparation according to any one of claims 8 to 11 for use in a method for treating a cancer patient or for the treatment of a patient suffering from a viral infection.
